# EUROPEAN PATENT APPLICATION

(11) **EP 0 945 422 A1**
(43) Date of publication of application: **29.09.1999**
(21) Application number: 97912427.8
(22) Date of filing: 12.11.1997
(51) Int. Cl.: C07C 43/225, C09K 19/12, C09K 19/14, C09K 19/30, C09K 19/42, G02F 1/13

(54) **PHENYL POLYHALOALKYL ETHER DERIVATIVES, LIQUID-CRYSTAL COMPOSITION CONTAINING THE SAME, AND LIQUID-CRYSTAL DISPLAY ELEMENT**

(30) Priority: 13.11.1996 JP 31695896
(71) Applicant: CHISSO CORPORATION, Osaka-shi Osaka 530-6591 (JP)
(72) Inventor: SHIBATA, Kouichi, Ichihara-shi, Chiba 290-0003 (JP); MATSUI, Shuichi, Ichihara-shi, Chiba 290-0035 (JP); MIYAZAWA, Kazutoshi, Yachiyo-shi, Chiba 276-0022 (JP); TAKEUCHI, Hiroyuki, Ichihara-shi, Chiba 290-0022 (JP); HISATSUNE, Yasusuke, Ichihara-shi, Chiba 299-0111 (JP); TAKESHITA, Fusayuki, Sodegaura-shi, Chiba 299-0261 (JP); NAKAGAWA, Etsuo, Chiba 290-0056 (JP)
(74) Representative: HOFFMANN - EITLE
(86) International application number: JP9704114
(87) International publication number: WO9821172

(57) **Abstract**

The invention is to provide novel liquid crystalline compounds exhibiting particularly high Δε, excellent miscibility with other liquid crystalline compounds, and nematic phase in a wide temperature range without impairing characteristics peculiar to liquid crystalline compounds having fluorine atom; to provide liquid crystal compositions comprising the compound; and to provide liquid crystal display devices fabricated by using the composition; the compounds are phenylpolyhaloalkyl ether derivatives expressed by the general formula (1) 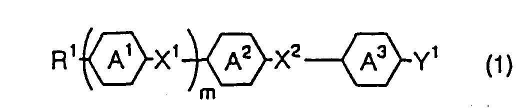 wherein X¹ and X² independently represent 1,2-ethylene group, 1,4-butylene group, or covalent bond provided that in no case are both X¹ and X² simultaneously a covalent bond; R¹ represents an alkyl group having 1 to 20 carbon atoms in which alkyl group methylene group may be replaced by oxygen atom, vinylene group, or ethynylene group; Y¹ represents -OCF₂CFH-(CF₂)ₙ-F (n is 0, 1, 2, or 3) or -OCF₂Cl; rings A¹ and A² independently represent 1,4-cyclohexylene ring, 1,4-cyclohexenylene ring, or 1,4-phenylene ring, A³ represents 1,4-phenylene ring, and the carbon atom in these rings may be replaced by oxygen atom or nitrogen atom, and the hydrogen atom on the rings may be replaced by fluorine atom or chlorine atom, respectively; m is 0 or 1; and each element which constitutes the compound may be its isotope.

## Description

### TECHNICAL FIELD

The present invention relates to liquid crystalline compounds and liquid crystal compositions. More specifically, the invention relates to phenylpolyhaloalkyl ether derivatives, novel liquid crystalline compounds which can develop preferable physical properties when used as electrooptical display material; to liquid crystal compositions comprising the derivative; and to liquid crystal display devices fabricated by using the liquid crystal composition.

### BACKGROUND ART

Liquid crystal display devices have been used for watches, tabletop calculators, various kind of measuring instruments, panels of automobiles, word processors, electronic notebooks, printers, computers, TV sets, or the likes. These commodity products utilize optical anisotropy and dielectric anisotropy among various physical properties of liquid crystalline compounds. As their displaying method, dynamic scattering (DS) type, guest · host (GH) type, twisted nematic (TN) type, super twisted nematic (STN) type, thin film transistor (TFT) type, and ferroelectric liquid crystal (FLC) are known. As driving mode, static driving mode, time shearing addressing mode, active matrix driving mode, and two-frequency addressing scheme are known.

Recently, liquid crystal display devices particularly having a higher display quality are required. In order to cope with the requirement, demand for display devices of active matrix mode represented, for example, by TFT type has been increased. Liquid crystal substances used for any display devices are required to be stable against moisture, air, heat, and light. Besides, the liquid crystal substances must exhibit a liquid crystal phase at temperatures in a range as wide as possible with room temperature being its center, and must have a low viscosity, good miscibility, high Δε, and most suitable Δn. However, no compounds are found at present which satisfy such requirements by a single compound, and thus it is a current situation that liquid crystal compositions produced by mixing several kind of liquid crystal compounds and liquid not-crystal compounds are used.

One of the characteristics required of TFT type liquid crystal display devices is to have a high contrast on display screen. Accordingly, liquid crystal substances used for this purpose are required to have a large specific resistivity, that is, to have a high voltage holding ratio (VHR) in addition to the requirements described above. Further, driving at a low voltage is required of TFT type liquid crystal display devices recently. In order to cope with this requirement, liquid crystalline compounds and liquid crystal compositions having a higher Δε than that of liquid crystal materials which were used for TFT type liquid crystal display devices until now became necessary.

Whereas many of general liquid crystalline compounds have cyano group, such compounds can not maintain a high specific resistivity during displaying since the compounds are decomposed when a high voltage is applied, in the case where such compounds are used in TFT type liquid crystal display devices. Accordingly, it is impossible to use liquid crystal compounds having cyano group for TFT type liquid crystal display devices despite the fact that the compounds have a high Δε. In order to improve this problem, development of liquid crystal materials having a high Δε while exhibiting a high specific resistivity has actively been conducted. As liquid crystal compounds having a high specific resistivity, fluorine compounds are suitable. These compounds are generally liquid crystalline ones having fluorine atom as substituent, and the following compounds are known as follows. For example, compounds expressed by the formula (10) are disclosed in Japanese Patent Publication No. Hei 02-40048.

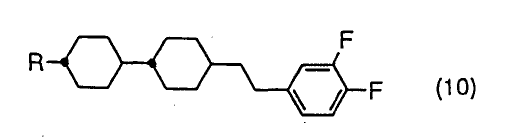

Whereas these compounds are used in a commercial scale since they have a high specific resistivity compared with compounds having cyano group, they have such a low Δε as 4 and are unable to realize a sufficiently low voltage driving.

As compounds having a higher Δε, trifluorophenyl compounds expressed by the following formula (11) are disclosed in Laid-open Japanese Patent Publication No. Hei 02-233626.

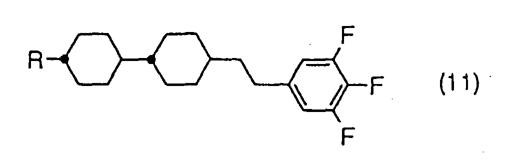

However, these compounds have defects such as Δε of the compounds are about 8 which is not necessarily high, temperature range of liquid crystal phase is very narrow, and clearing point is low since the compounds have a structure in which more fluorine atoms than those of the compounds of the formula (10) described above are introduced (for instance, it is confirmed that clearing point of the compounds of the formula (11) becomes lower by about 60°C and by about 25°C compared with 1-(4-propylcyclohexyl)-4-{2-(4-fluorophenyl)ethyl}cyclohexane which have the same structure as that of the formula (11) with the exception that one fluorine atom is introduced instead of three fluorine atoms introduced at the right side terminal, and 1-(4-propylcyclohexyl)-4-{2-(3,4-difluorophenyl)ethyl}cyclohexane having the same structure as that of the formula (11) with the exception that two fluorine atoms are introduced as in the same way as described above, respectively).

Further, fluorine compounds expressed by the formula (12) or (13) are disclosed in Laid-open Japanese Patent Publication No. 04-506361.

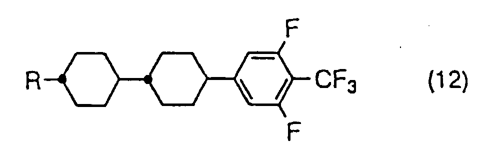

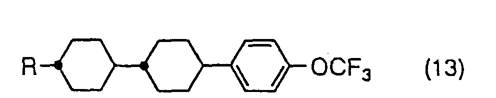

Whereas these compounds have a comparatively high Δε (for example, Δε of the compounds of the formula (13) is about 7), miscibility with existing liquid crystalline compounds is considerably poor particularly at low temperatures, and thus the compounds are not suitable as component of liquid crystal compositions.

In order to improve this miscibility, compounds in which fluorine atom is introduced in the alkyl group R are disclosed in WO Japanese Patent Publication (Tokuhyo) No. Hei 04-506817. Whereas the disclosed compounds are two or three-rings compounds having cyclohexyl group and phenyl group at terminals, they still can not sufficiently improve the miscibility. As an example, the compound expressed by the formula (14) is disclosed. However, this compound has only single bond as bonding group in the center portion of the molecule, and does not have other bonding groups, for example, 1,2-ethylene group. Besides, the substituent on the terminal phenyl group is limited to fluorine atom, and the publication does not include any description or suggestion about other substituents, for example, fluoroalkyl group and fluoroalkoxy group. In addition, the compound of the formula (14) does not exhibit even a liquid crystal phase.

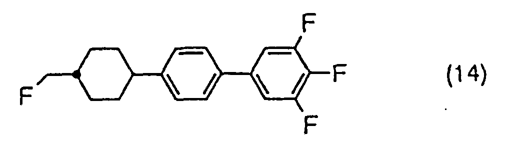

As described above, since compounds having a high Δε and being excellent in miscibility have not yet been found, it is impossible to increase the mixing ratio of the liquid crystalline compounds having a high Δε when they are used as component of liquid crystal compositions. As the result, it is a current situation that the height of Δε of liquid crystal compositions is limited.

Accordingly, appearance of liquid crystalline compounds excellent in miscibility with existing liquid crystalline compounds while having a high specific resistivity and a high Δε are expected.

### DISCLOSURE OF THE INVENTION

An object of the present invention is to solve the defects in the conventional technology. Another object of the invention is to provide liquid crystalline compounds which improve three characteristics in such ways as described below without impairing general properties (viscosity is low (response speed is high), stability is high, and optical anisotropy value (Δn) is appropriately large) of liquid crystalline compounds having fluorine atom; to provide liquid crystal compositions comprising the compound; and to provide liquid crystal display devices fabricated by using the composition.
1) Dielectric anisotropy value (Δε) is large.
2) Miscibility with other liquid crystalline compounds is excellent.
3) Temperature range in which the compounds exhibit nematic phase is wide.

As a result of diligent investigation by the present. inventors, it has been found out that liquid crystalline compounds having polyhaloalkyloxy group at a terminal and having 1,2-ethylene group or 1,4-butylene group as a bonding group in the center portion of the molecule exhibit remarkably excellent characteristics, leading to the accomplishment of the present invention.

That is, the present invention is summarized as follows:
(1) A phenylpolyhaloalkyl ether derivative expressed by the general formula (1) 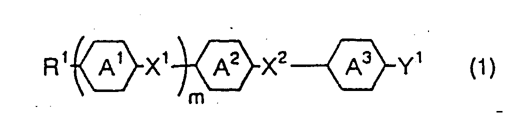 wherein X¹ and X² independently represent 1,2-ethylene group, 1,4-butylene group, or covalent bond provided that in no case are both X¹ and X² simultaneously a covalent bond; R¹ represents an alkyl group having 1 to 20 carbon atoms in which alkyl group methylene group may be replaced by oxygen atom, vinylene group, or ethynylene group; Y¹ represents -OCF₂CFH-(CF₂)ₙ-F (n is 0, 1, 2, or 3) or -OCF₂Cl; rings A¹ and A² independently represent 1,4-cyclohexylene ring, 1,4-cyclohexenylene ring, or 1,4-phenylene ring, A³ represents 1,4-phenylene ring, and the carbon atom in these rings may be replaced by oxygen atom or nitrogen atom, and the hydrogen atom on the rings may be replaced by fluorine atom or chlorine atom, respectively; m is 0 or 1; and each element which constitutes the compound may be its isotope.
(2) The phenylpolyhaloalkyl ether derivative described in paragraph (1) above wherein m is 0, and ring A² is 1,4-cyclohexylene ring.
(3) The phenylpolyhaloalkyl ether derivative described in paragraph (1) above wherein m is 1, ring A¹ is 1,4-cyclohexylene ring, and X² is a covalent bond.
(4) The phenylpolyhaloalkyl ether derivative described in paragraph (1) above wherein m is 1, and X¹ and X² are independently 1,2-ethylene group or 1,4-butylene group.
(5) The phenylpolyhaloalkyl ether derivative described in paragraph (3) above wherein ring A² is 2-fluoro-1,4-phenylene group (in which the carbon atom at position 1 links to ring A³) or 2,6-difluoro-1,4-phenylene group (in which the carbon atom at position 1 links to ring A³).
(6) The phenylpolyhaloalkyl ether derivative described in paragraph (3) above wherein ring A² is 1,4-cyclohexylene group.
(7) A liquid crystal composition comprising at least one phenylpolyhaloalkyl ether derivative described in any one of paragraphs (1) to (6) above.
(8) A liquid crystal composition comprising, as a first component, at least one phenylpolyhaloalkyl ether derivative described in any one of paragraphs (1) to (6) above, and comprising, as a second component, at least one compound selected from the group consisting of the compounds expressed by any one of the general formulas (2), (3), and (4) 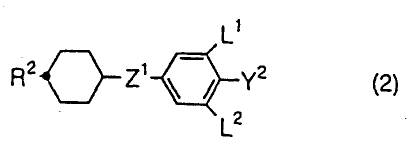 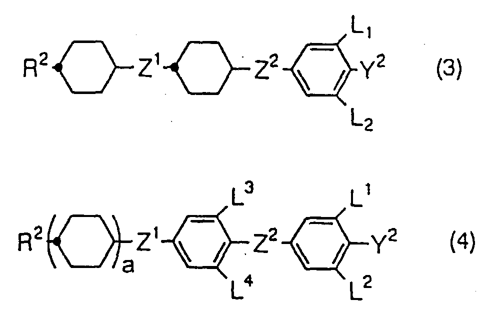 wherein R² represents an alkyl group having 1 to 10 carbon atoms; Y² represents fluorine atom, chlorine atom, trifluoromethoxy group, difluoromethoxy group, trifluoromethyl group, difluoromethyl group, or monofluoromethyl group; L¹, L², L³, and L⁴ independently represent hydrogen atom or fluorine atom; Z¹ and Z² independently represent 1,2-ethylene group, vinylene group, or a covalent bond; and a is 1 or 2.
(9) A liquid crystal composition comprising, as a first component, at least one phenylpolyhaloalkyl ether derivative described in any one of paragraphs (1) to (6) above, and comprising, as a second component, at least one compound selected from the group consisting of the compounds expressed by any one of the general formulas (5), (6), (7), (8), (9) 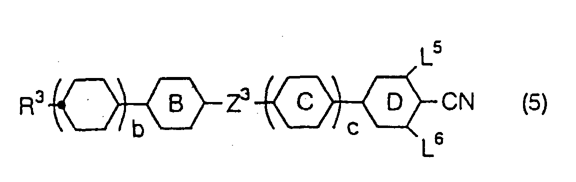 wherein R³ represents fluorine atom, an alkyl group having 1 to 10 carbon atoms, or an alkenyl group having 2 to 10 carbon atoms in which alkyl group or alkenyl group one or not adjacent two or more methylene groups may be represented by oxygen atom; ring B represents 1,4-cyclohexylene, 1,4-phenylene, or 1,3-dioxane-2,5-diyl: ring C represents 1,4-cyclohexylene, 1,4-phenylene, or pyrimidine-2,5-diyl; ring D represents 1,4-cyclohexylene or 1,4-phenylene; Z³ represents 1,2-ethylene group, oxycarbonyl group, or a covalent bond; L⁵ and L⁶ independently represent hydrogen atom or fluorine atom: and b and c are independently 0 or 1, 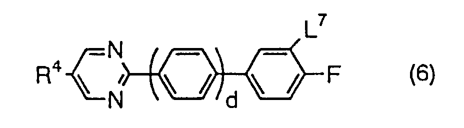 wherein R⁴ represents an alkyl group having 1 to 10 carbon atoms; L⁷ represents hydrogen atom or fluorine atom; and d is 0 or 1, 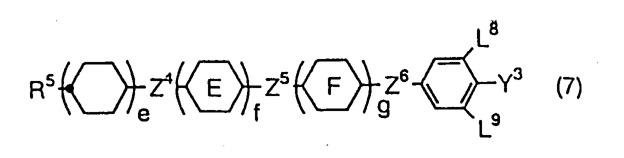 wherein R⁵ represents an alkyl group having 1 to 10 carbon atoms; ring E and ring F independently represent 1,4-cyclohexylene or 1,4-phenylene; Z⁴ and Z⁵ independently represent oxycarbonyl group or a covalent bond; Z⁶ represent oxycarbonyl group or ethynylene group; L⁸ and L⁹ independently represent hydrogen atom or fluorine atom; Y³ represents fluorine atom, trifluoromethoxy group, difluoromethoxy group, trifluoromethyl group, difluoromethyl group, or monofluoromethyl group; and e, f, and g are independently 0 or 1, 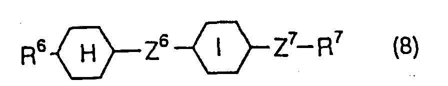 wherein R⁶ and R⁷ independently represent an alkyl group having 1 to 10 carbon atoms or an alkenyl group having 2 to 10 carbon atoms in which alkyl or alkenyl group one or not-adjacent two or more methylene groups may be replaced by oxygen atom; ring H represents 1,4-cyclohexylene, 1,4-phenylene, or pyrimidine-2,5-diyl; ring I represents 1,4-cyclohexylene or 1,4-phenylene; Z⁶ represents ethynylene group, oxycarbonyl group, 1,2-ethylene group, 1-butene-3-ynylene group, or a covalent bond; and Z⁷ represents oxycarbonyl group or a covalent bond, 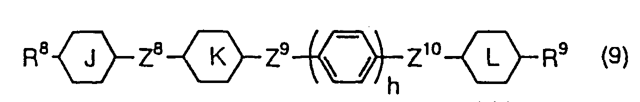 wherein R⁸ and R⁹ independently represent an alkyl group having 1 to 10 carbon atoms or an alkenyl group having 2 to 10 carbon atoms in which alkyl or alkenyl group one or not-adjacent two or more methylene groups may be replaced by oxygen atom; ring J represents 1,4-cyclohexylene, 1,4-phenylene, or pyrimidine-2,5-diyl; ring K represents 1,4-cyclohexylene, 1,4-phenylene in which one or more hydrogen atoms may be replaced by fluorine atom, or pyrimidine-2,5-diyl; ring L represents 1,4-cyclohexylene or 1,4-phenylene; Z⁸ and Z¹⁰ independently represent oxycarbonyl group, 1,2-ethylene group, or covalent bond; Z⁹ represents vinylene group, ethynylene group, oxycarbonyl group, or a covalent bond; and h is 0 or 1.
(10) A liquid crystal composition comprising, as a first component, at least one phenylpolyhaloalkyl ether derivative described in any one of paragraphs (1) to (6) above, and comprising, as a part of a second component, at least one compound selected from the group consisting of the compounds expressed by any one of the general formulas (2), (3), and (4) described in paragraph (8) above, and comprising, as another part of the second component, at least one compound selected from the group consisting of the compounds expressed by any one of the general formulas (5), (6), (7), (8), and (9) described in paragraph (9) above.
(11) A liquid crystal display device fabricated by using the liquid crystal composition described in any one of paragraphs (7) to (10) above.

### BEST MODE FOR CARRYING OUT THE INVENTION

Phenylpolyhaloalkyl ether derivatives, liquid crystalline compounds of the present invention expressed by the general formula (1) are characterized principally in that the compounds have a structure in which 1,4-phenylene ring and side chain (Y¹), -OCF₂CFH-(CF₂)ₙ-F (n is 0, 1, 2, or 3) or -OCF₂Cl linking to the ring exist at a terminal. On account of such structure, the compounds have a high Δε and an excellent miscibility at the same time, exhibit a low viscosity and high stability (high specific resistivity). Besides, most of the compounds come to have a wide temperature range of liquid crystal phase and to be extremely stable chemically.

Since the liquid crystalline compounds of the present invention expressed by the general formula (1) exhibit excellent characteristics and have a good miscibility (in other words, have a high solubility to other liquid crystalline compounds and liquid crystal compositions) as described above, the liquid crystal compositions produced by using the liquid crystalline compound of the present invention as their component do not lose nematic phase at low temperatures, for example, at -20°C which is required from the aspect of practical use.

Also, since they have a low viscosity, even when they are used in a large amount as component of liquid crystal compositions, viscosity of the whole liquid crystal compositions does not particularly rise, and the extent to which viscosity rises is small even at low temperatures since dependency of viscosity on temperature is extremely small and stability of the viscosity is high. Accordingly, the liquid crystalline compounds of the present invention are useful as component especially when liquid crystal compositions having a high response speed are produced.

Further, since they are extremely stable chemically, it is possible to make the specific resistivity and voltage holding ratio of liquid crystal compositions extremely high by using the compounds as component, and practically usable liquid crystal compositions stable against such external factors as UV light and heat can be provided.

As described above, in the general formula (1), while R¹ can extensively represents an alkyl group having 1 to 20 carbon atoms, or an alkoxy group, alkoxyalkyl group, alkenyl group, or alkynyl group having 2 to 20 carbon atoms,
as the alkyl group; methyl group, ethyl group, n-propyl group, n-butyl group, n-pentyl group, n-hexyl group, n-heptyl group, and n-octyl group,
as the alkoxy group; methoxy group, ethoxy group, n-propoxy group, n-butoxy group, n-pentyloxy group, and n-hexyloxy group,
as the alkoxyalkyl group; methoxymethyl group, ethoxymethyl group, n-propoxymethyl group, n-butoxymethyl group, n-pentyloxymethyl group, n-hexyloxymethyl group, methoxyethyl group, ethoxyethyl group, n-propoxyethyl group, n-butoxyethyl group, n-pentyloxyethyl group, methoxypropyl group, ethoxypropyl group, n-propoxypropyl group, n-butoxypropyl group, methoxybutyl group, ethoxybutyl group, n-propoxybutyl group, methoxypentyl group, ethoxypentyl group, and methoxyhexyl group,
as the alkenyl group; vinyl group, 1-propenyl group, 2-propenyl group, 1-butenyl group, 2-butenyl group, 3-butenyl group, 1-pentenyl group, 2-pentenyl group, 3-pentenyl group, 4-pentenyl group, 1-hexenyl group, 2-hexenyl group, 3-hexenyl group, 4-hexenyl group, 5-hexenyl group, 1-heptenyl group, 2-heptenyl group, 3-heptenyl group, 4-heptenyl group, 5-heptenyl group, 6-heptenyl group, 1-octenyl group, 2-octenyl group, 3-octenyl group, 4-octenyl group, 5-octenyl group, 6-octenyl group, and 7-octenyl group, and
as alkynyl group; ethynyl group, 1-propynyl group, 2-propynyl group, 1-butynyl group, 2-butynyl group, 3-butynyl group, 1-pentynyl group, 2-pentynyl group, 3-pentynyl group, 4-pentynyl group, 1-hexynyl group, 2-hexynyl group, 3-hexynyl group, 4-hexynyl group, 5-hexynyl group, 1-heptynyl group, 2-heptynyl group, 3-heptynyl group, 4-heptynyl group, 5-heptynyl group, 6-heptynyl group, 1-octynyl group, 2-octynyl group, 3-octynyl group, 4-octynyl group, 5-octynyl group, 6-octynyl group, and 7-octynyl group
can preferably be mentioned, respectively.

Rings A¹, A², and A³ are also as described above. As the rings in which carbon atom in the ring is replaced by oxygen atom or nitrogen atom, pyran ring, 1,3-dioxane ring, and 1,4-dioxane ring, preferably 1,3-dioxane ring for the former; and pyridine ring, pyrimidine ring, pyrazine ring, pyridazine ring, triazine ring, tetrazine ring, and piperidine ring, preferably pyridine ring and pyrimidine ring for the latter can be mentioned, respectively.

It is needless to say that 1,4-cyclohexylene ring, 1,4-cyclohexenylene ring, and 1,4-phenylene ring in all of which carbon atom in the ring is not replaced by oxygen atom or nitrogen atom are preferable rings.

Each element which constitute the compounds expressed by the general formula (1) may be selected from its isotopes. This is because a substantial difference is not brought about in the characteristics of liquid crystals by such fact.

It is possible to make the Δn and Δε value of the compounds of the present invention more desired largeness by selecting proper substituents with reference to the Y¹, R¹, rings A¹, A², and A³ described above, and X¹, X², and m already mentioned above, and using them in proper combination.

While the compounds of the present invention are preferable as component of liquid crystal compositions particularly for TFT, they are also efficient liquid crystalline compounds as component of liquid crystal compositions for other applications, for examples, for TN, STN, gust-host mode, polymer dispersion type liquid crystal display devices, dynamic scattering, or FLC.

Liquid crystal compositions provided according to the present invention comprise, as a first component, at least one liquid crystalline compound expressed by the general formula (1).

Its content is necessary to be 0.1 to 99.9 % by weight based on the weight of liquid crystal composition to develop excellent characteristics, and the content is preferably 1 to 50 % by weight, and more desirably 3 to 20 % by weight.

While the liquid crystal compositions of the present invention may comprise only the first component described above, compositions in which at least one compound (hereinafter referred to as second component A) selected from the group consisting of the compounds expressed by one of the general formulas (2), (3), and (4) described above and/or at least one compound (hereinafter referred to as second component B) selected from the group consisting of the compounds expressed by one of the general formulas (5) to (9) described above is mixed as a second component is preferable. Further, an optically active compound, and known compounds used for the purpose of adjusting threshold voltage, temperature range of liquid crystal phase, Δn, Δε, or viscosity, can be mixed.

Among the second component A mentioned above,
as preferable examples of the compounds included in the general formula (2), the compounds expressed by one of the following formulas (2-1) to (2-15),
as preferable examples of the compounds included in the general formula (3), the compounds expressed by one of the formulas (3-1) to (3-48), and
as preferable examples of the compounds included in the general formula (4), the compounds expressed by one of the formulas (4-1) to (4-53)
can be mentioned, respectively. 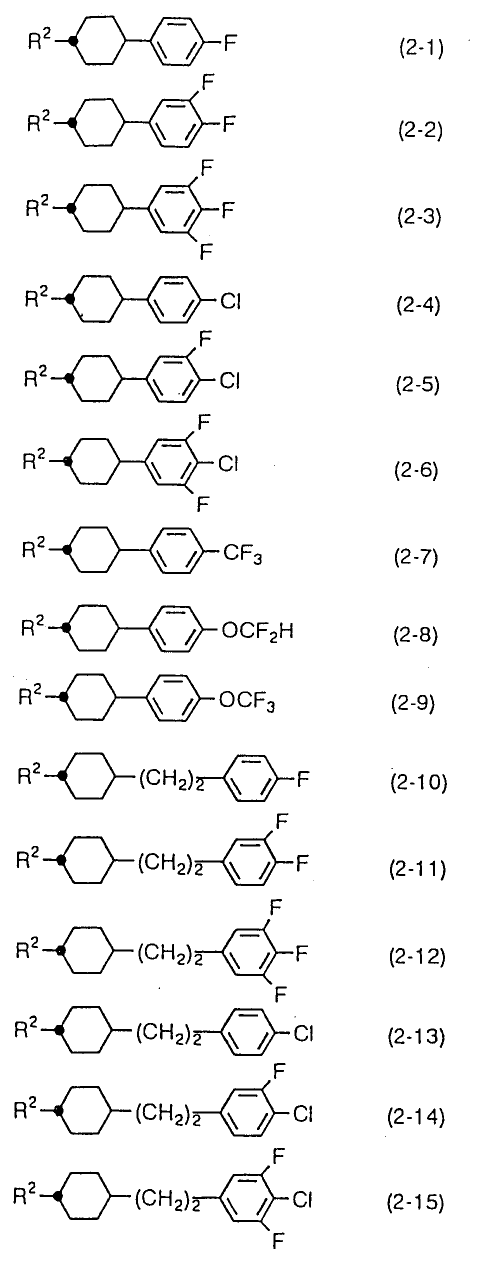 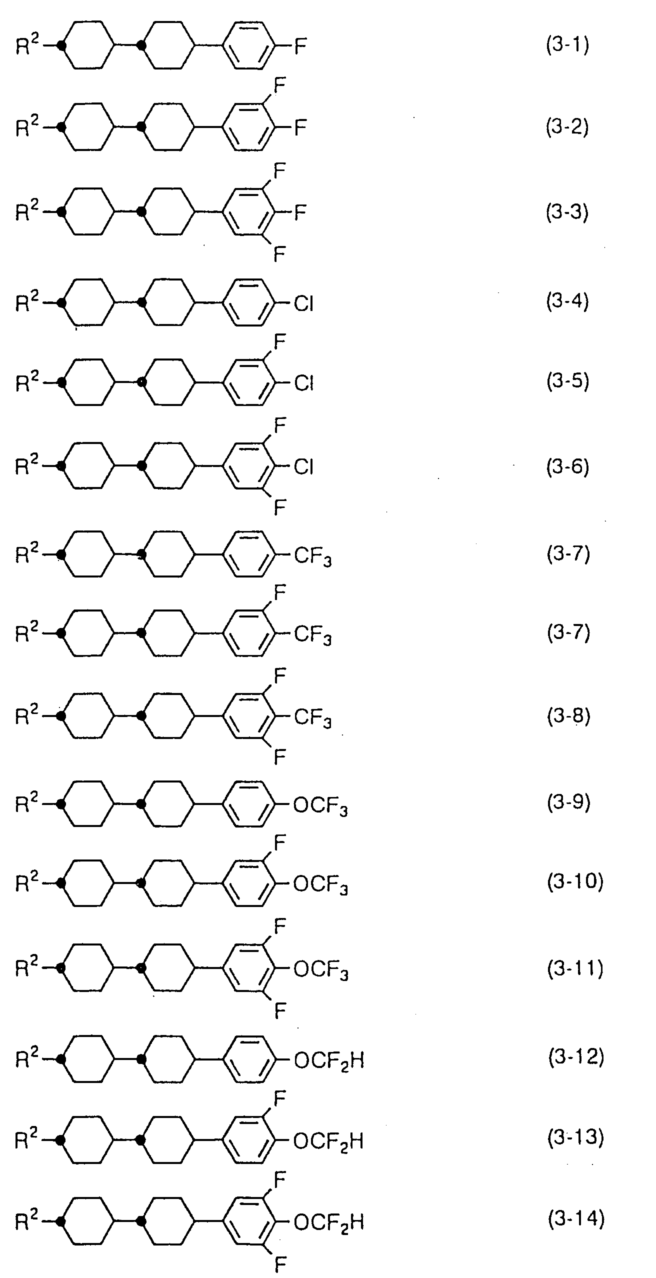 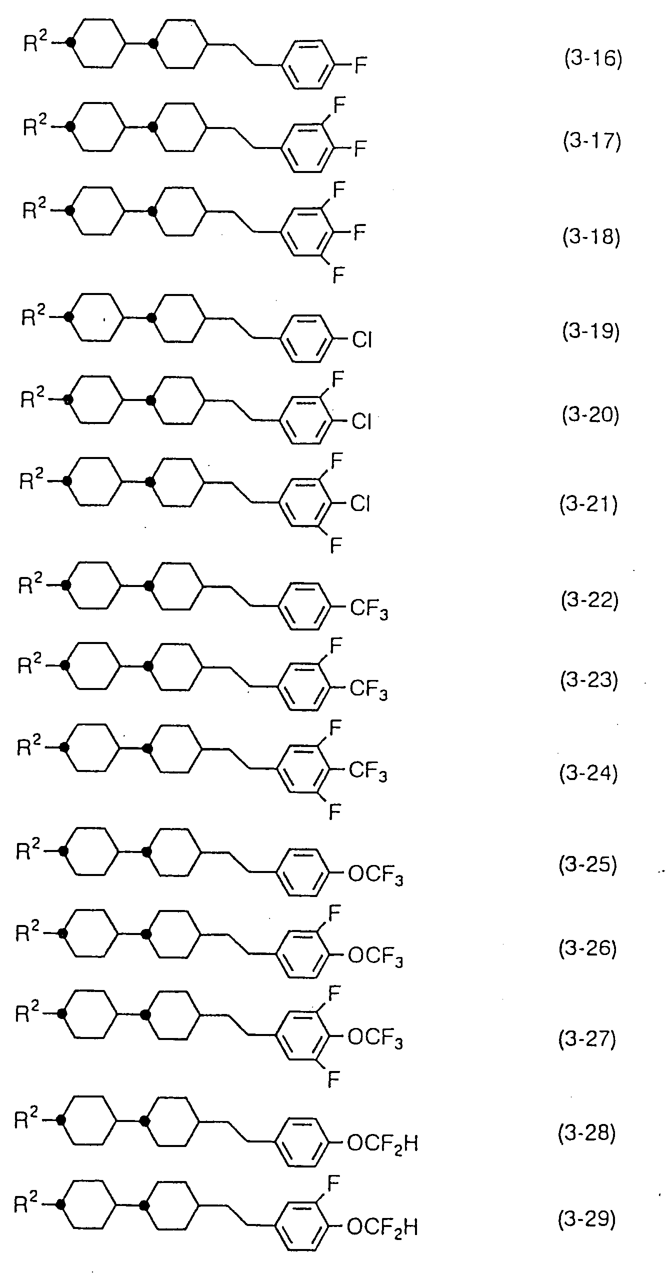 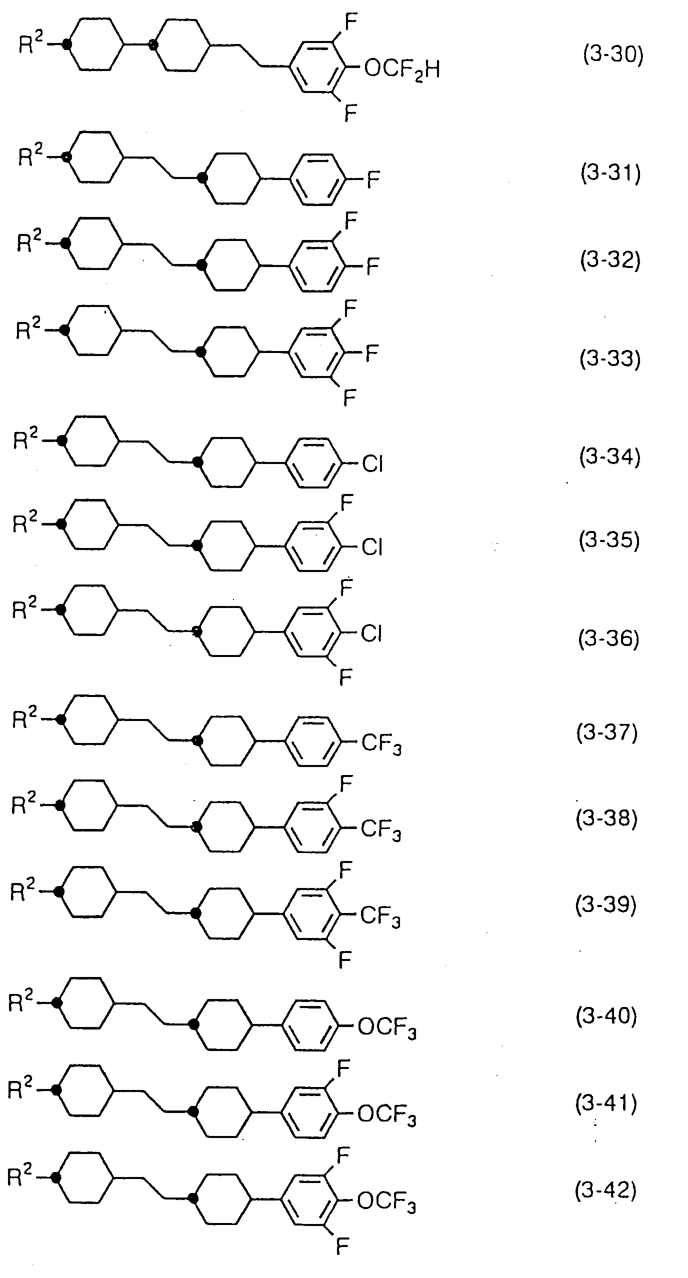 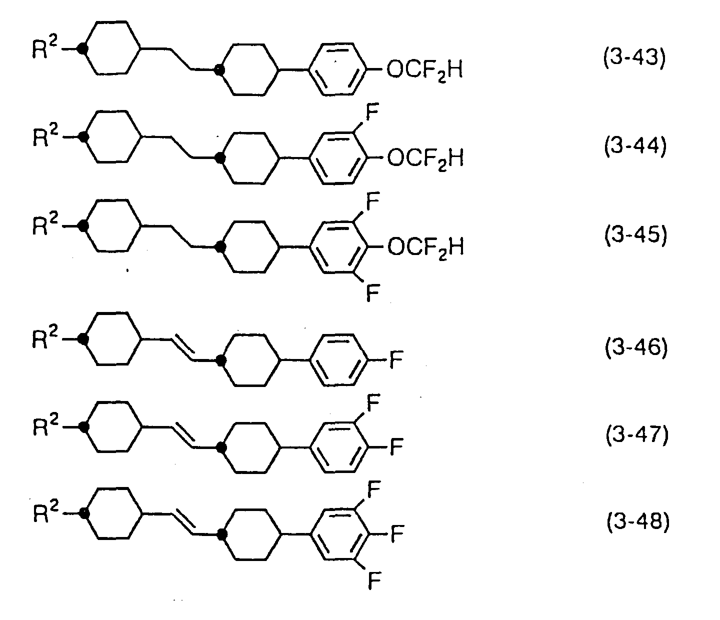 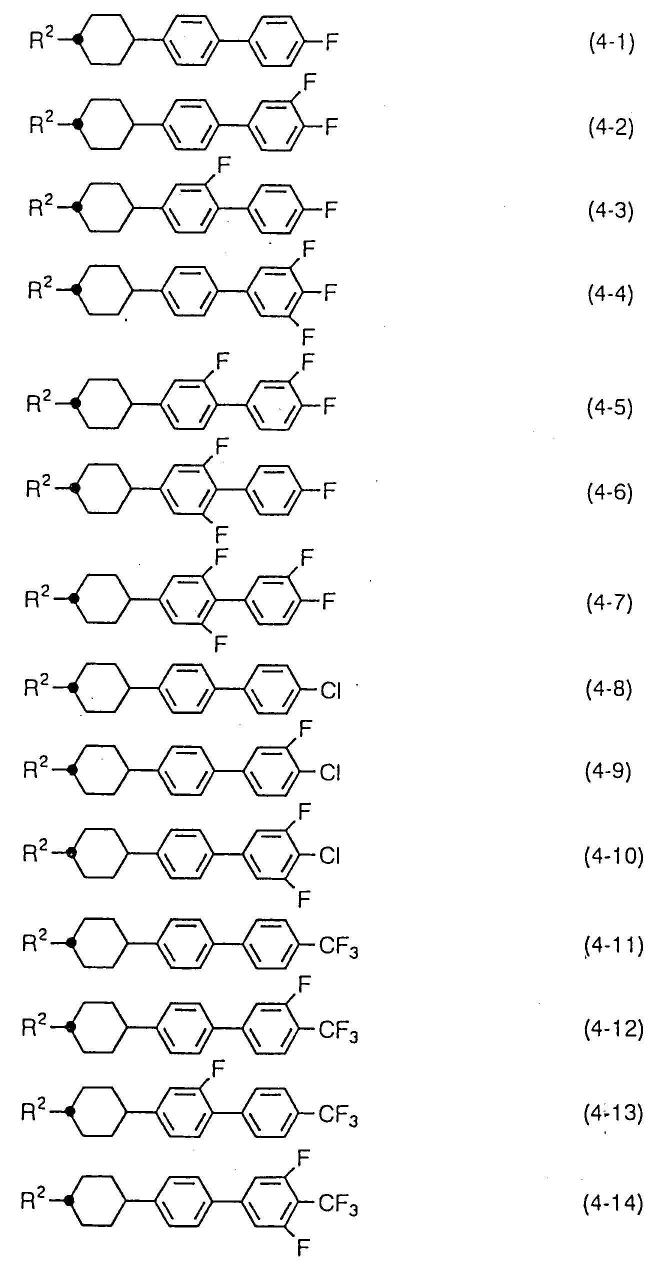 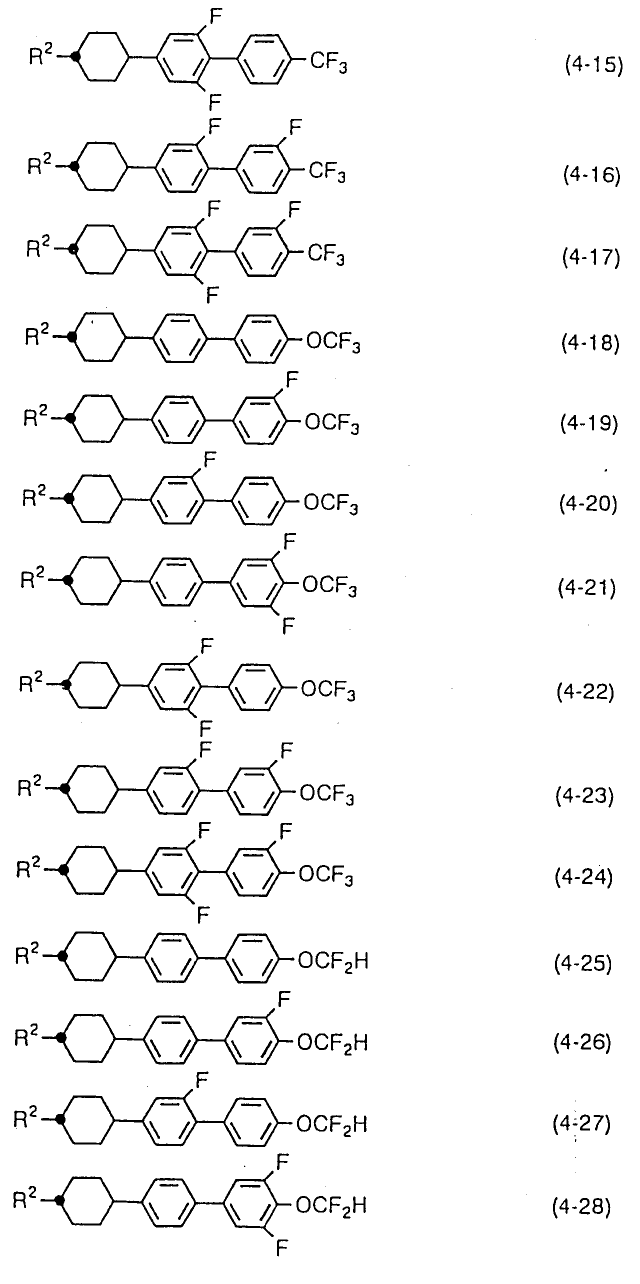 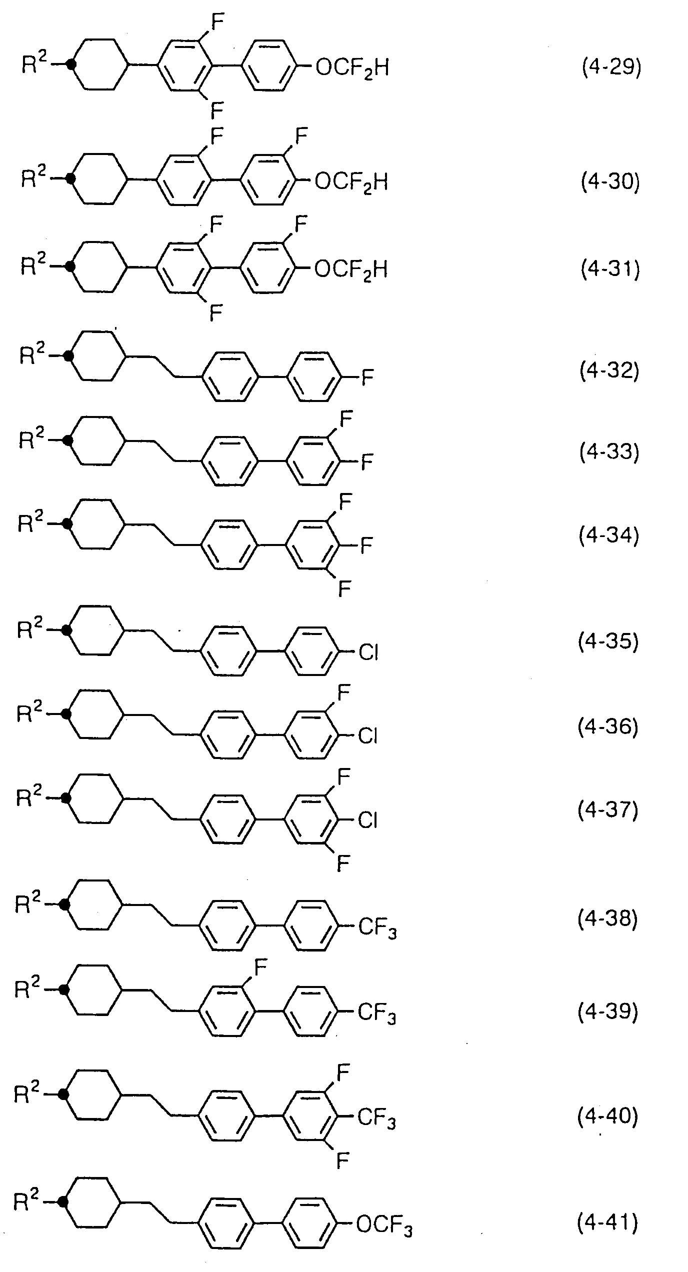 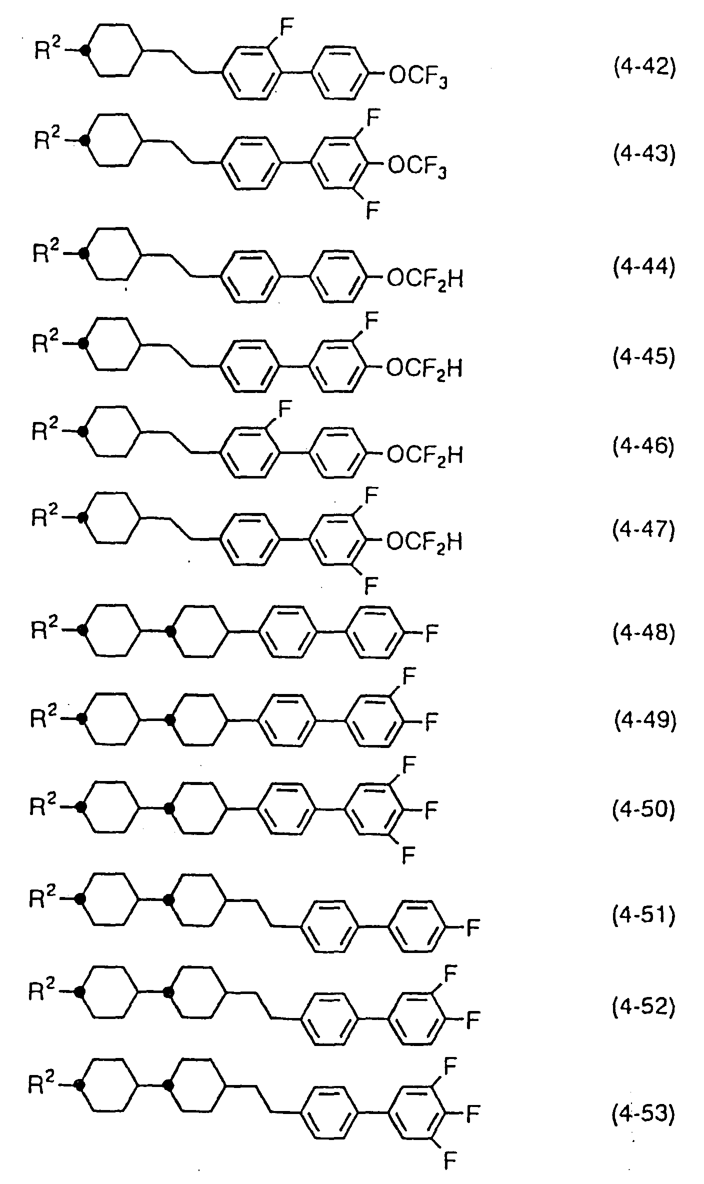 wherein R² have the same meaning as described above.

Compounds expressed by one of these general formulas (2) to (4) exhibit a positive Δε, are excellent in thermal stability and chemical stability, and thus are indispensable when liquid crystal compositions for TFT are produced of which a high reliability such as a high voltage holding ratio (large specific resistivity) is required.

The amount of the compounds to be used is suitably in the range of 1 to 99 % by weight based on the total amount of liquid crystal composition when liquid crystal compositions for TFT are produced, and the amount is preferably 10 to 97 % by weight and more desirably 40 to 95 % by weight. In that case, a compound expressed by one of the general formulas (5) to (9) may be comprised as a part of the components,

Compounds expressed by one of the general formulas (2) to (4) described above can also be used when liquid crystal compositions for STN display mode or ordinary TN display mode are produced.

Next, among the second component B,
as preferable examples of the compounds included in the general formula (5), the compound of the following formulas (5-1) to (5-24),
as preferable examples of the compounds included in the general formula (6), the compounds of the following formulas (6-1) to (6-3), and
as preferable examples of the compounds included in the general formula (7), the compounds of the following formulas (7-1) to (7-28)
can be mentioned, respectively. 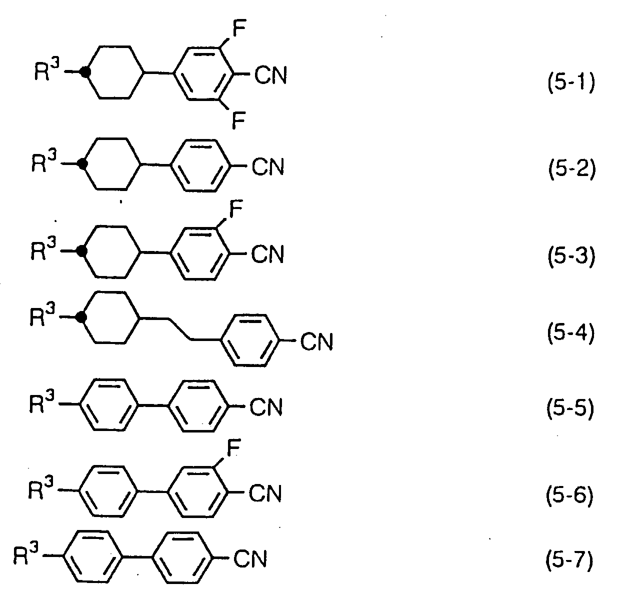 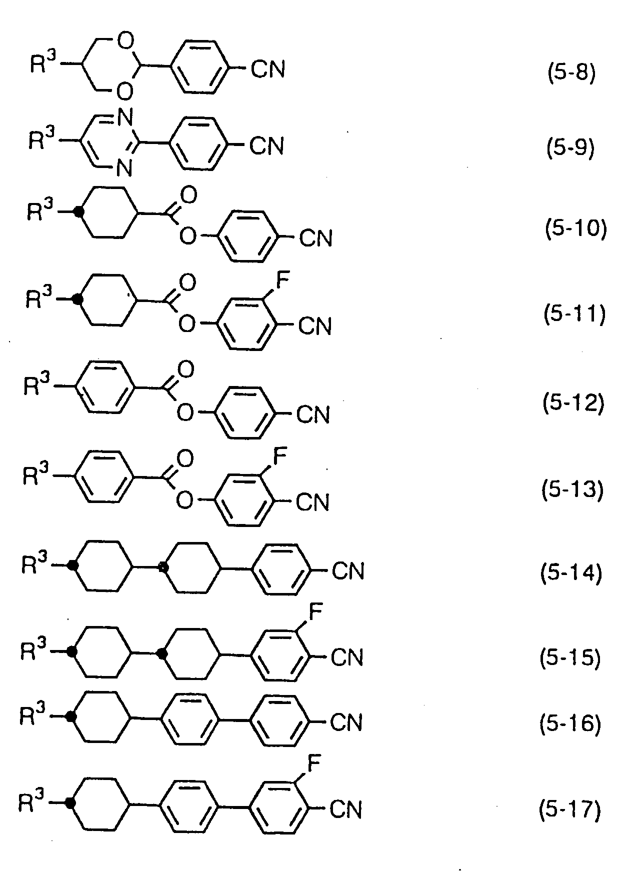 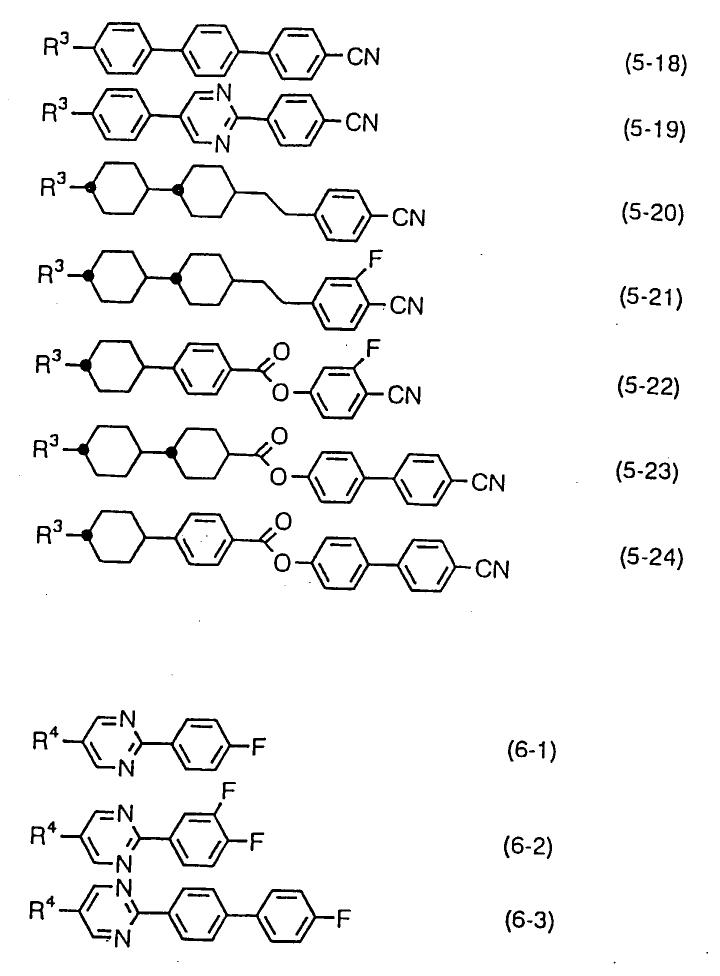 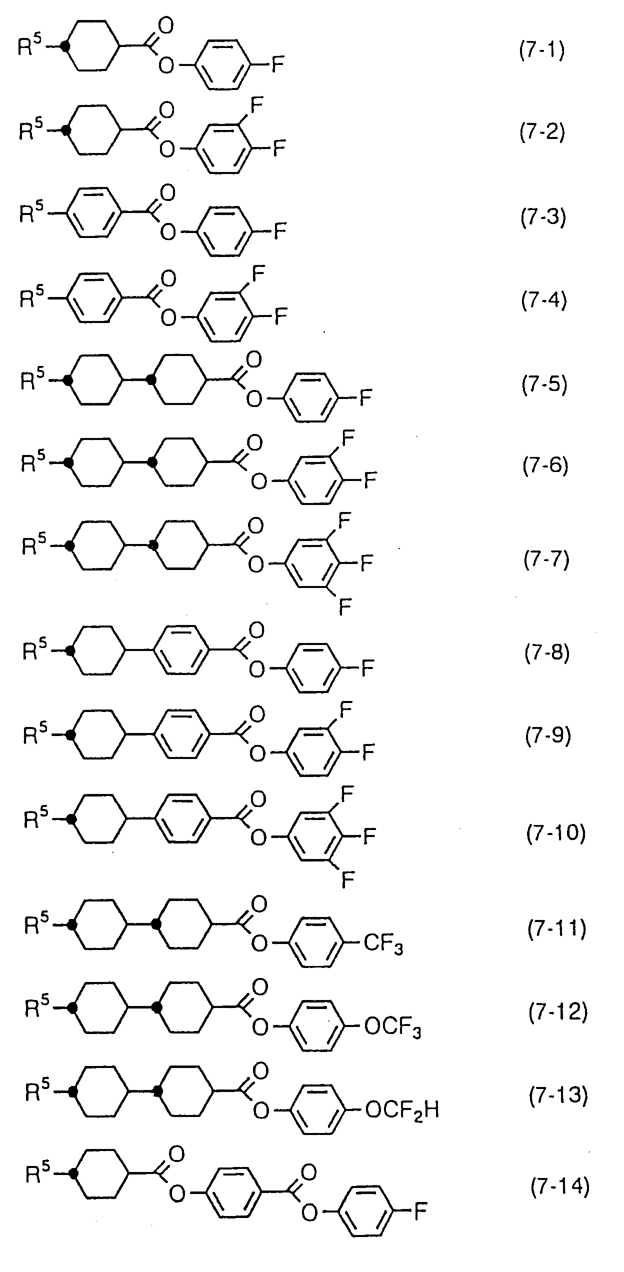 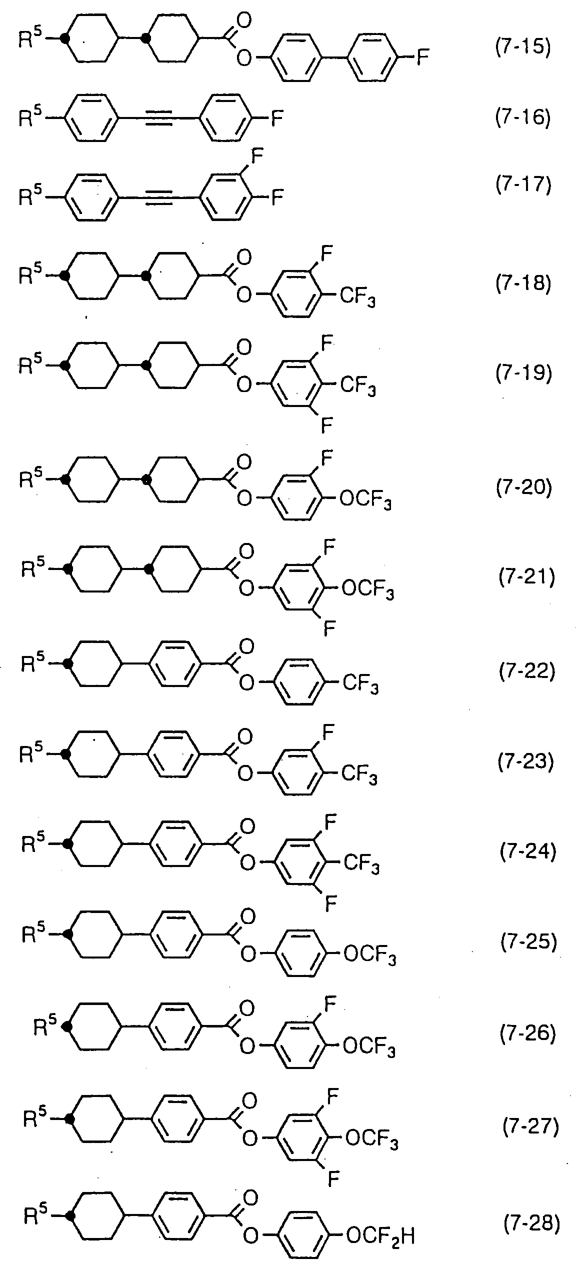 wherein R³ to R⁵ have the same meaning as described above.

Compounds expressed by one of these general formulas (5) to (7) have a positive and large Δε value, and are used particularly for the purpose of lowering threshold voltage of liquid crystal compositions. Also, they are used for the purpose of adjusting viscosity and Δn, for the purpose of widening nematic range such as raising clearing point, and for the purpose of improving the steepness of threshold voltage.

Among the second component B described above,
as preferable examples of the compounds included in the general formulas (8), the compounds of the following formulas (8-1) to (8-8), and
as preferable examples of the compounds included in the general formula (9), the compounds of the following formulas (9-1) to (9-13)
can be mentioned, respectively. 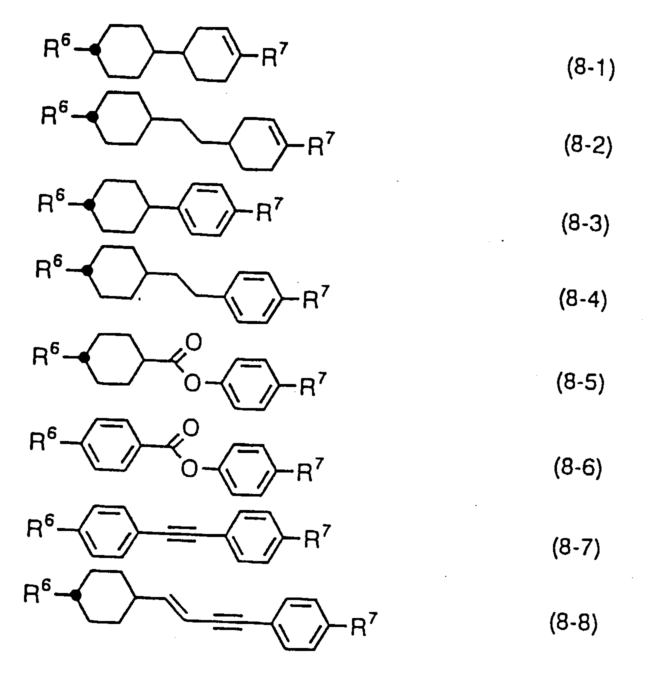 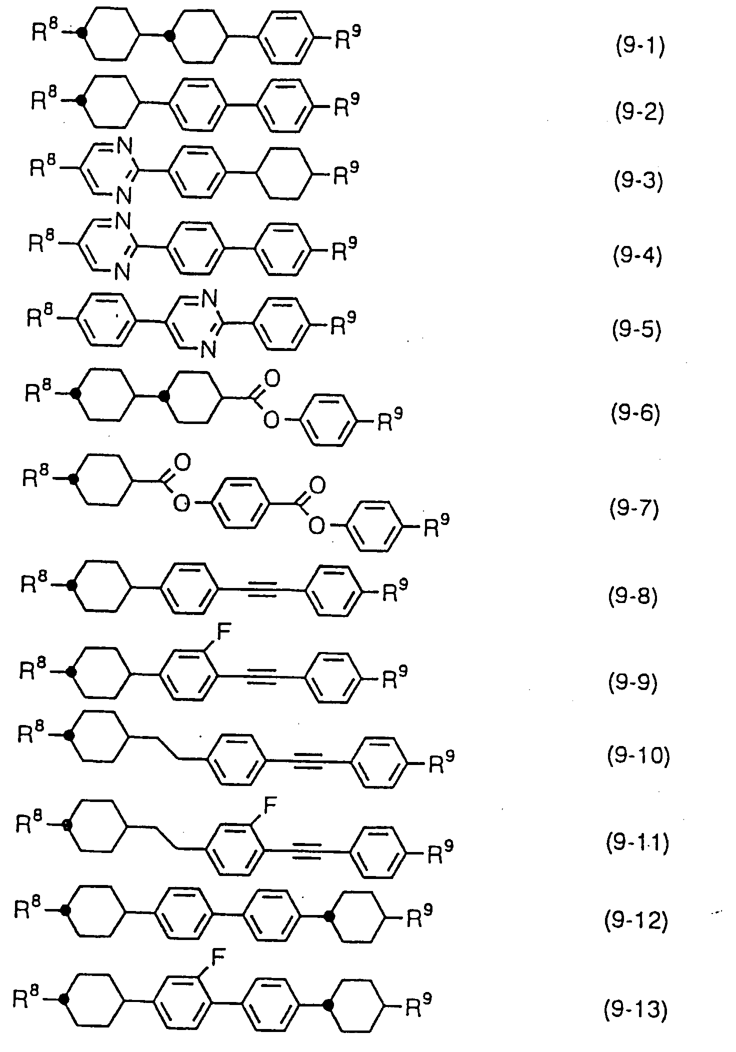 wherein R⁶ to R⁹ have the same meaning as described above.

Compounds expressed by one of the general formula (8) or (9) have a negative or slightly positive Δε, the compounds of the former formula are used principally for the purpose of reducing viscosity or adjusting Δn, and compounds of the latter formula are used for the purpose of widening nematic range such as raising clearing point or for the purpose of adjusting Δn.

The second component B described above are indispensable compounds particularly when liquid crystal compositions for STN display mode or TN display mode are produced.

Its amount to be used is suitably in the range of 1 to 99 % by weight based on the total amount of liquid crystal composition when liquid crystal compositions for ordinary TN display mode, or STN display mode are produced, and the amount is preferably 10 to 97 % by weight and more desirably 40 to 95 % by weight. In that case, compounds expressed by one of the general formulas (2) to (4) may be used as a part of components.

Liquid crystal compositions provided according to the present invention can be produced by methods which are conventional by themselves. For instance, they are produced by a method in which various components are dissolved with each other at a high temperature and under a reduced pressure, or by a method in which various components are dissolved in an organic solvent, mixed, and then the solvent is distilled off under a reduced pressure.

Also, when necessary, improvements are performed and optimized depending on the intended applications by adding a suitable additive. Such additive is well known in the art and described in the literature in detail. Usually, a chiral dopant or the like having an effect of inducing helical structure of liquid crystal to adjust required twist angle and to prevent reverse twist.

When a dichroic dye such as merocyanine type, styryl type, azo type, azomethine type, azoxy type, quinophthalone type, anthraquinone type, and tetrazine type is added, the liquid crystal compositions of the present invention can be used as ones for guest-host (GH) mode.

Liquid crystal compositions of the present invention can be used as ones for electrically controlled birefringence (ECB) mode or dynamic scattering (DS) mode, including as NCAP which is prepared by the microencapsulation of a nematic liquid crystal, or as liquid crystal compositions for polymer dispersed liquid crystal display devices (PDLCD) represented by polymer net work liquid crystal display devices (PNLCD) prepared by forming a polymer of three-dimensional reticulated structure in a liquid crystal.

Liquid crystalline compounds of the present invention expressed by the general formula (1) can readily be produced by using known chemical procedures of organic synthesis, for example, the procedures described in Organic Synthesis, Organic Reactions, or Jikken Kagaku Kouza (Course of Chemical Experiment) (Maruzen), or the likes in proper combination.

Preparation of the liquid crystalline compounds expressed by the general formula (1) wherein ring A² is an aromatic ring and X² is a covalent bond:

After aryl iodide (15) prepared by a known method is lithiated to obtain lithium reagent (16), it is converted into arylboric acid (17), and then this acid can be subjected to a coupling with aryl bromide (18) to obtain compound (1) which is an example of the compounds of the present invention.

In the reactions described above, lithiation of aryl iodide (15) can be performed by quite general procedures, for instance, by a method in which n-butyl lithium, sec-butyl lithium, or ter-butyl lithium (hereinafter collectively referred to as BuLi) is used (Shin Jikken Kagaku Kouza (Course of New Chemical Experiment), Vol. 12, pp 57-58), a method in which metal lithium (Li) is used (Shin Jikken Kagaku Kouza, Vol. 12, pp 53-54), or a method in which magnesium is used in place of metal lithium to obtain a Grignard reagent (Shin Jikken Kagaku Kouza, Vol. 12, 00 68-69).

Conversion into arylboric acid (17) can be performed by reacting triisopropyl borate or trimethyl borate with the lithium reagent or Grignard reagent (16) obtained by the method described above, and then hydrolysing the product in an acidic condition (J. Org. Chem., 49, 5237 (1984)).

For the coupling of the arylboric acid (17) with aryl bromide (18), it is sufficient to conduct reaction in the presence of sodium carbonate and a catalytic amount of palladium (0) (Org. Syn. Chem., 51, 1043 (1993)).

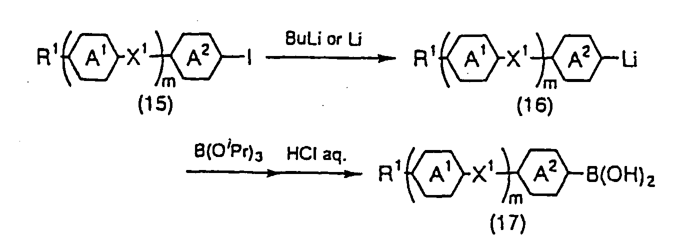

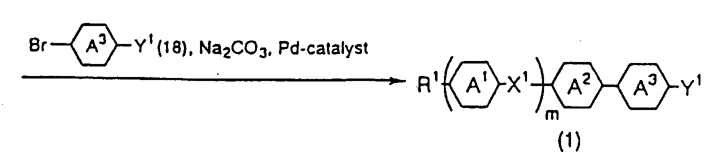

### Preparation of the compounds expressed by the general formula (1) wherein ring A² is a not-aromatic ring and X² is a covalent bond:

After aryl iodide (18) is lithiated to convert into lithium reagent (19) in the same manner as described above, it is added to a ketone (20) to obtain alcohol (21), and then the alcohol can be subjected to a dehydration and hydrogenation to obtain compound (1), an example of the compounds of the present invention.

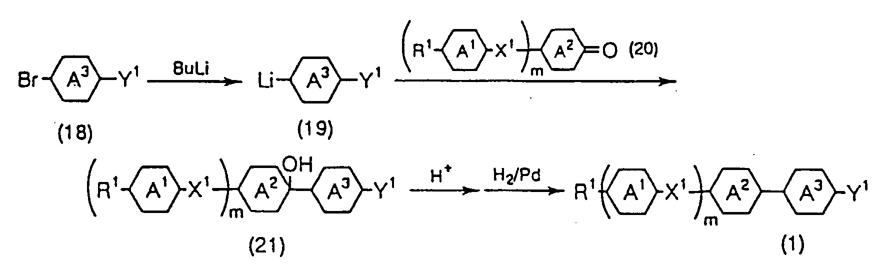

### Preparation of the compounds expressed by the general formula (1) wherein ring A² is an aromatic ring and X² is a not-covalent bond:

Lithium reagent (16) mentioned above and 1,2-dibromoethane or 1,4-dibromobutane (22) are reacted to obtain compound (23), and this compound can be reacted with lithium reagent (19) to obtain compound (1), an example of the compounds of the present invention.

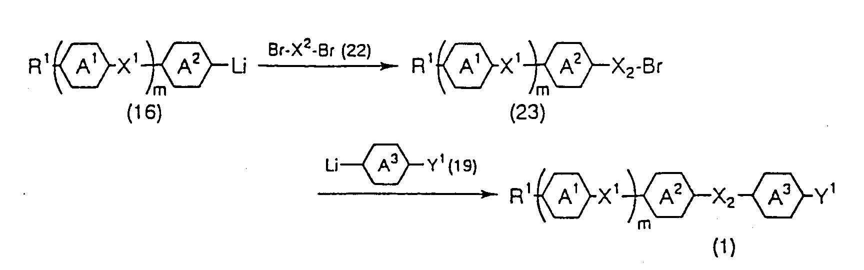

### Preparation of the compounds expressed by the general formula (1) wherein ring A² is a not-aromatic ring and X² is a not-covalent bond:

Lithium reagent (19) is reacted with 1,2-dibromoethane or 1,4-dibrmobutane (22) to obtain compound (24), and then the compound is reacted with triphenylphosphine to obtain Wittig reagent (25). This reagent can be reacted with the ketone (20) mentioned above in the presence of a base to convert the reagent into olefin derivative (26) and then hydrogenated to obtain compound (1), an example of the compounds of the present invention.

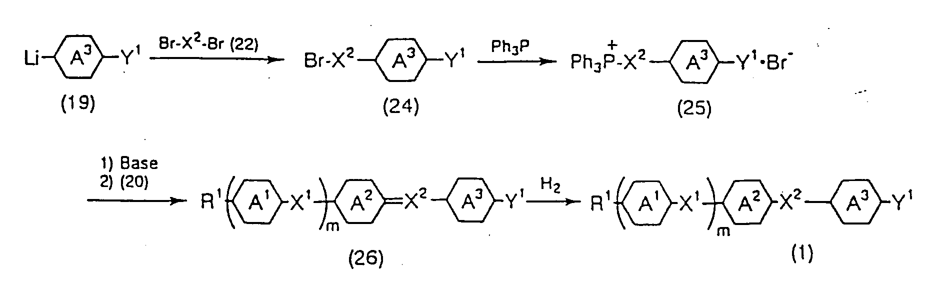

Now, the present invention will be described in more detail with reference to Examples. However, it should be understood that the scope of the present invention is by no means restricted by such specific Examples.

In each of the Examples, C indicates crystal, N: nematic phase, S: smectic phase, and I: isotropic liquid, and the unit of all phase transition temperatures is °C.

### Example 1

### Preparation of 4-(1,1,2,3,3,3-hexafluoropropyloxy)-4'-(2-(trans-4-pentylcyclohexyl)ethyl)-2',3,5-trifluorobiphenyl (Compound expressed by the general formula (1) wherein R¹ = n-pentyl group, A¹ = 1, 4-cyclohexylene ring, X¹ = 1,4-butylene group, A² = 2-fluoro-1,4-phenylene group, X² = a covalent bond, A³ = 2,6-difluoro-1,4-phenylene group, Y¹ = -OCF₂CFHCF₃, (Compound No. 99))

To 72 ml of a solution of 9.66 g (24 mmol) of 2-fluoro-4-(2-(trans-4-(pentylcyclohexyl)ethyl))iodobenzene in ether was added by drops 15.8 ml (1.60M; 25.2 mmol) of n-butyl lithium in hexane at -78°C under nitrogen gas atmosphere over 20 minutes. One hour later, the solution of aryl lithium thus obtained was added by drops to 24 ml of a solution of 9.03 g (48 mmol) of triisopropyl borate in ether at -78°C under nitrogen gas atmosphere over 20 minutes. After the solution was stirred as it was for 21 hours while being gradually raised up to room temperature, the solution was again cooled down to 0°C, and 30 ml of 3M hydrochloric acid was added by drops thereto over 5 minutes. After stirring for 10 minutes, it was raised back to room temperature. Then, water and ether were added to the solution, the solution was separated into layers, the extraction from the water layer was conducted thrice with ether, and the organic layer was combined and dried over magnesium sulfate. The magnesium sulfate was filtered off, and the filtrate was concentrated under a reduced pressure to obtain 4.02 g of a crude product.

This product was washed with heptane to obtain 5.20 g of 2-fluoro-4-(2-(trans-4-(pentylcyclohexyl)ethyl))phenyl boric acid. Yield was 67 %.

Mixed solution of 2.82 g (8.8 mmol) of the boric acid thus obtained, 2.87 g (8 mmol) of 3,5-difluoro-4-(1,1,2,3,3,3-hexafluoropropyloxy)bromobenzene, 1.70 g (16 mmol) of sodium carbonate, and 0.462 g (0.4 mmol) of tetrakis(triphenylphosphine)palladium (0) in 16 ml of toluene and 8 ml of water was heated to reflux under nitrogen gas atmosphere for 10 hours. After the solution was cooled down to room temperature, water and toluene were added thereto, the solution was filtered through Celite, the filtrate was separated into layers, and then the extraction from the water layer was conducted thrice with toluene. The organic layer was combined and dried over magnesium sulfate. The magnesium sulfate was filtered off, the filtrate was concentrated under a reduced pressure to obtain 4.95 g of a residue. This residue was purified by silica gel column chromatography (eluent: heptane) to obtain 4.32 g of while crystals. Yield was 97 %. Further, the crystals were recrystallized from mixed solvent of 10 ml of heptane and 3 ml of Solmix to obtain 2.75 g of the subject compound, 4-(1,1,2,3,3,3-hexafluoropropyloxy)-4'-(2-(trans-4-pentylcyclohexyl)ethyl)-2',3,5-trifluorobiphenyl. Yield was 62 %.
C 41.4 N 99.5 I

### Example 2

### Preparation of the compound expressed by the general formula (1) wherein R¹ = n-pentyl group, A¹, A² = 1,4-cyclohexylene group, X¹ = 1,4-butylene group, X² = a covalent bond, A³ = 2-fluoro-1,4-phenylene group, Y¹ = -OCF₂CF₂H (Compound No. 100)

To 24 ml of a solution of 3.67 g (12 mmol) of 3-fluoro-4-(1,1,2,2-tetrafluoroethoxy)bromobenzene in ether was added by drops 7.9 ml (1.60M; 12.6 mmol) of a solution of n-butyl lithium in hexane at -78°C over 20 minutes. After it was stirred at the same temperature for 10 minutes, 24 ml of a solution of 3.34 g (12 mmol) of 4-(2-(trans-4-(pentylcyclohexyl)ethyl)cyclohexanone in ether was added thereto. The solution was raised up to 0°C in 4 hours, and then saturated aqueous ammonium chloride solution was added thereto to terminate the reaction. Further, water and toluene were added thereto, separated into layers, the extraction from the water layer was conducted thrice with toluene, the organic layer was combined and dried over magnesium sulfate. After the magnesium sulfate was filtered off, the filtrate was concentrated under a reduced pressure, and the 6.12 g of the residue thus obtained was purified by silica gel column chromatography to obtain 5.10 g of an intermediate additive. Yield was 86 % and the ratio of isomers was 1.7 : 1.

A toluene solution in an amount of 31 ml in which 5.10 g (10.4 mmol) of the additive and 0.1 g (0.52 mmol) of p-toluenesulfonic acid·monohydrate were added was heated to reflux for 1 hour. The reaction solution was cooled down to room temperature, washed with saturated aqueous sodium bicarbonate solution, and then magnesium sulfate was added thereto to dry the solution. After the magnesium sulfate was filtered off, the filtrate was concentrated under a reduced pressure, 5.03 g of the residue thus obtained was purified by silica gel column chromatography (eluent: heptane) to obtain 4.45 g of 3-fluoro-1-(4-(2-(trans-4-pentylcyclohexyl)ethyl)cyclohexenyl)-4-(1,1,2,2-tetrafluoroethoxy)benzene. Yield was 91 %.

To a solution of 4.45 g (9.4 mmol) of this product in 31 ml of toluene and 31 ml of Solmix was added 0.223 g of palladium-carbon (palladium content: 5 % by weight), and then stirred under hydrogen gas atmosphere at room temperature under a normal pressure for 4 hours. After the catalyst was filtered off, the filtrate was concentrated under a reduced pressure to obtain 4.51 g of a crude product. Yield was 100 %. The ratio of isomers was trans:cis = 1.6:1.

The crude product was recrystallized from mixed solvent of heptane-ethanol to obtain 0.7 g of the subject compound comprising only trans isomer. Yield was 16 %.
C 68.7 N 141.6 I

Based on the description in Examples 1 and 2, and section of BEST MODE FOR CARRYING OUT THE INVENTION, the following compounds, Compound Nos. 1 through 193 can be prepared. In the following, each compound is designated by using each of the symbols used in the general formula (1), they are indicated by symbols R¹, A², X², A³, and Y¹ in the case of m = 0; and indicated by symbols R¹, A¹, X¹, A², X², A³, and Y¹ in the case of m = 1. In the following, compounds obtained in Example 1 or 2 are shown again.

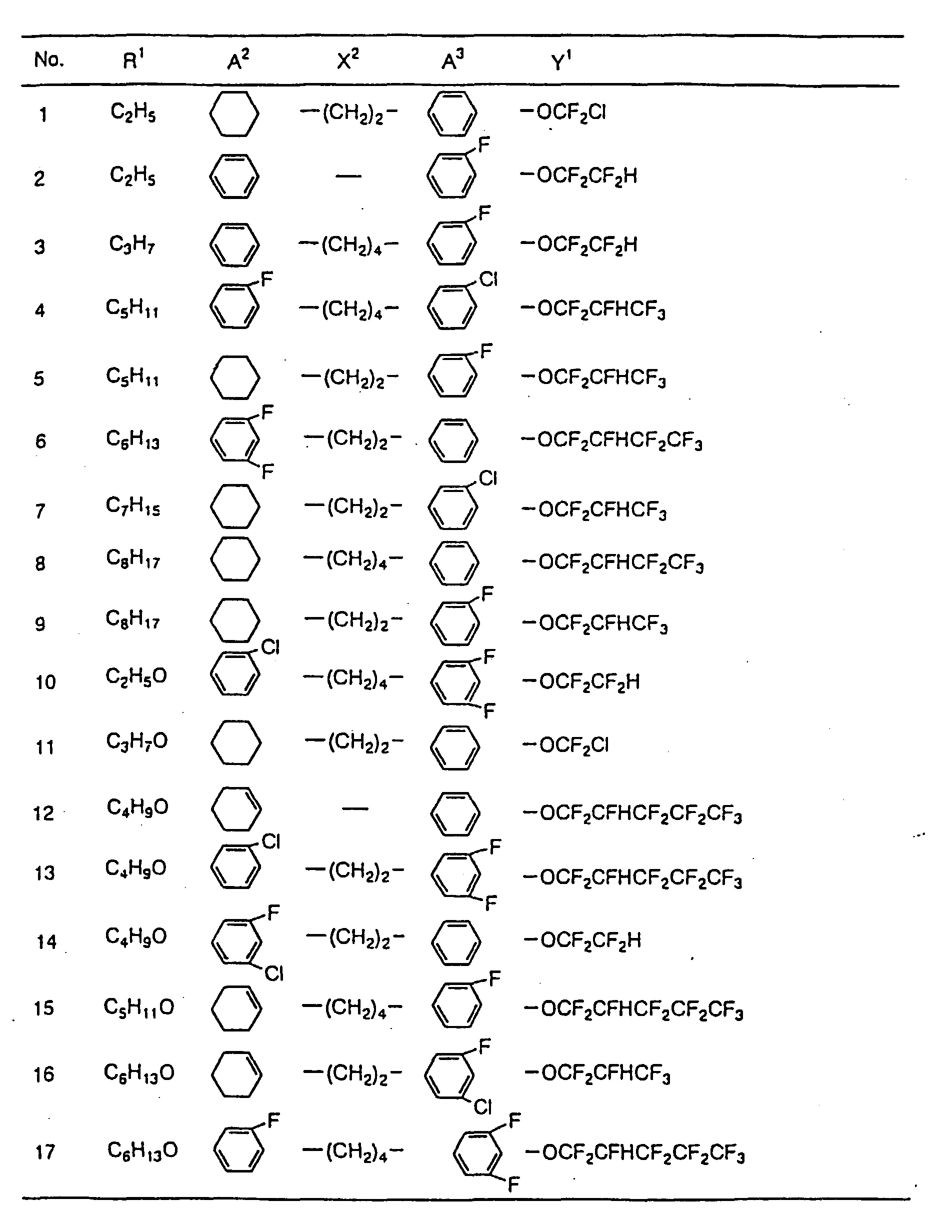

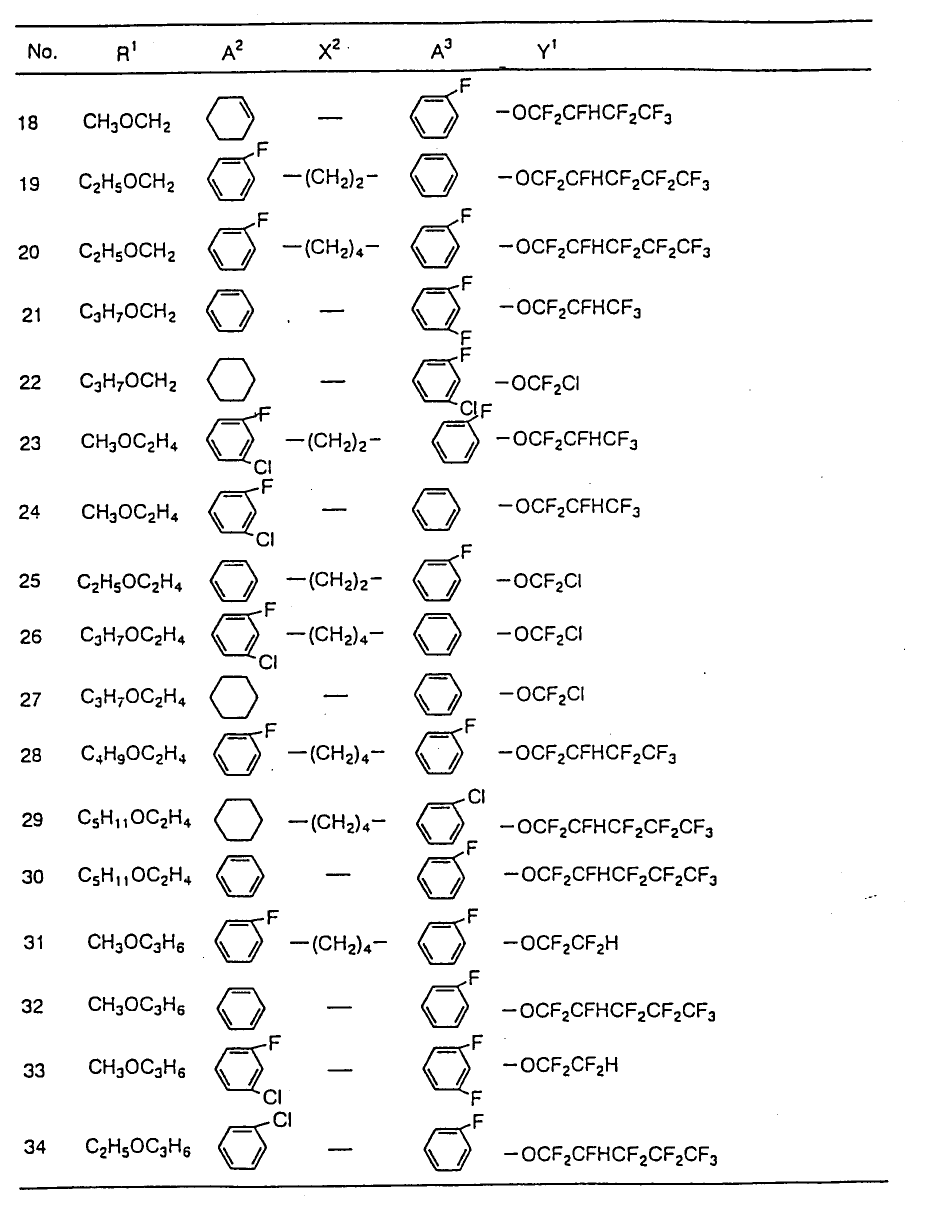

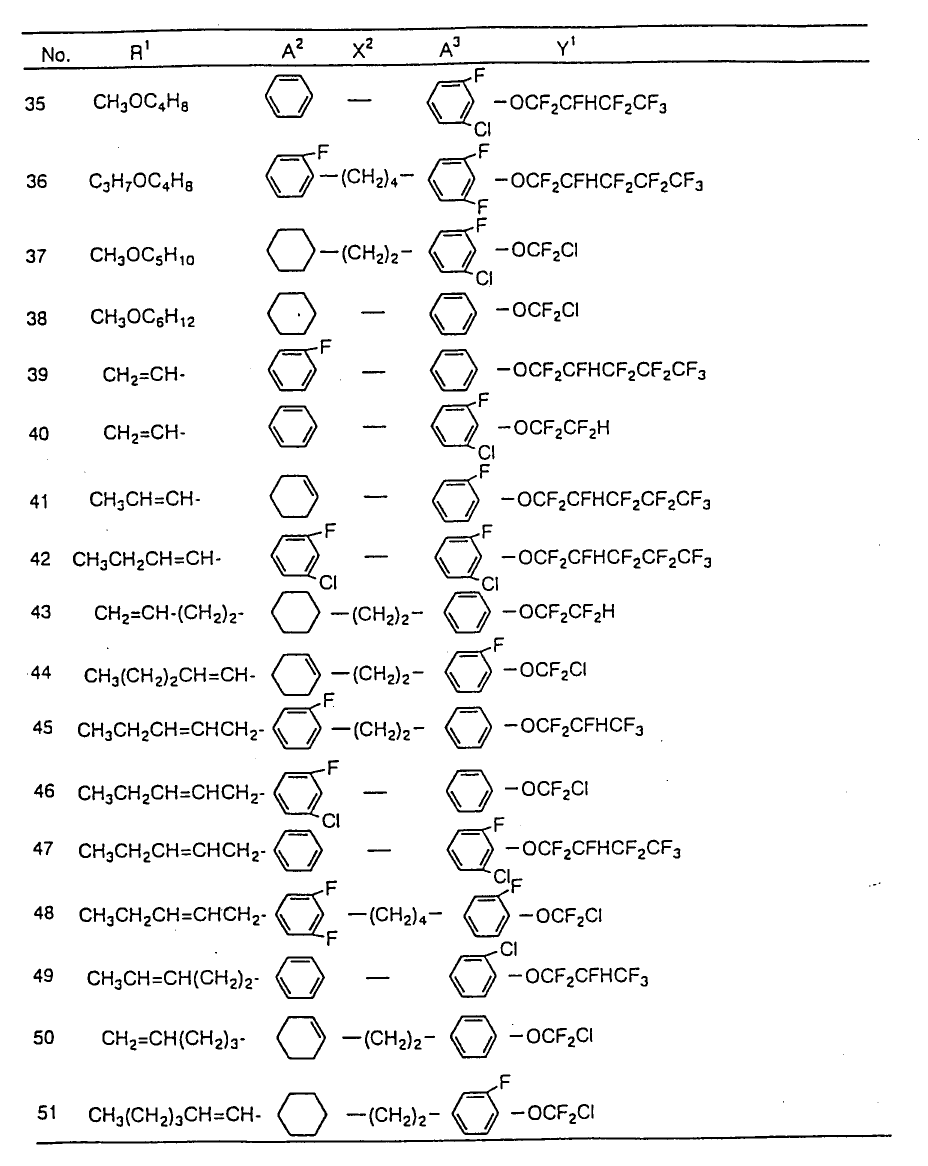

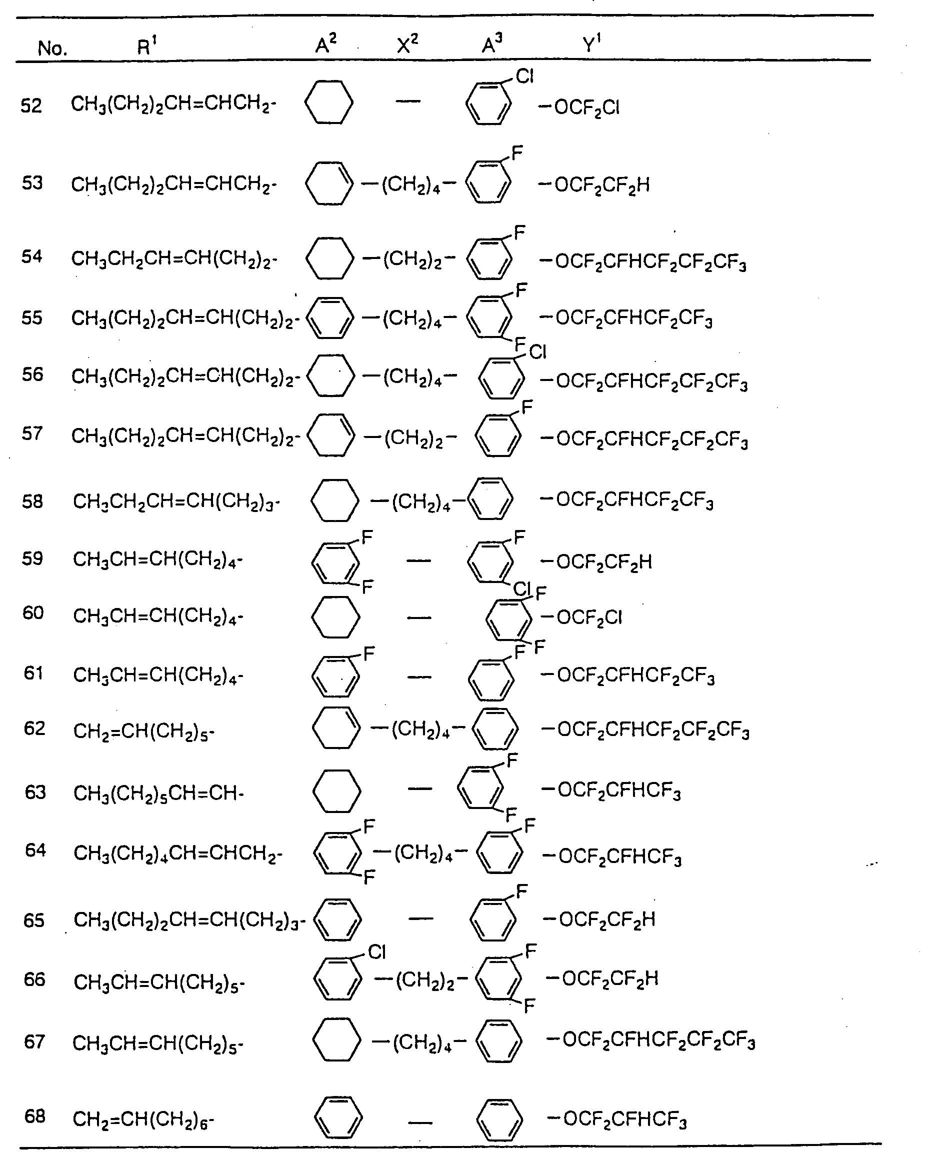

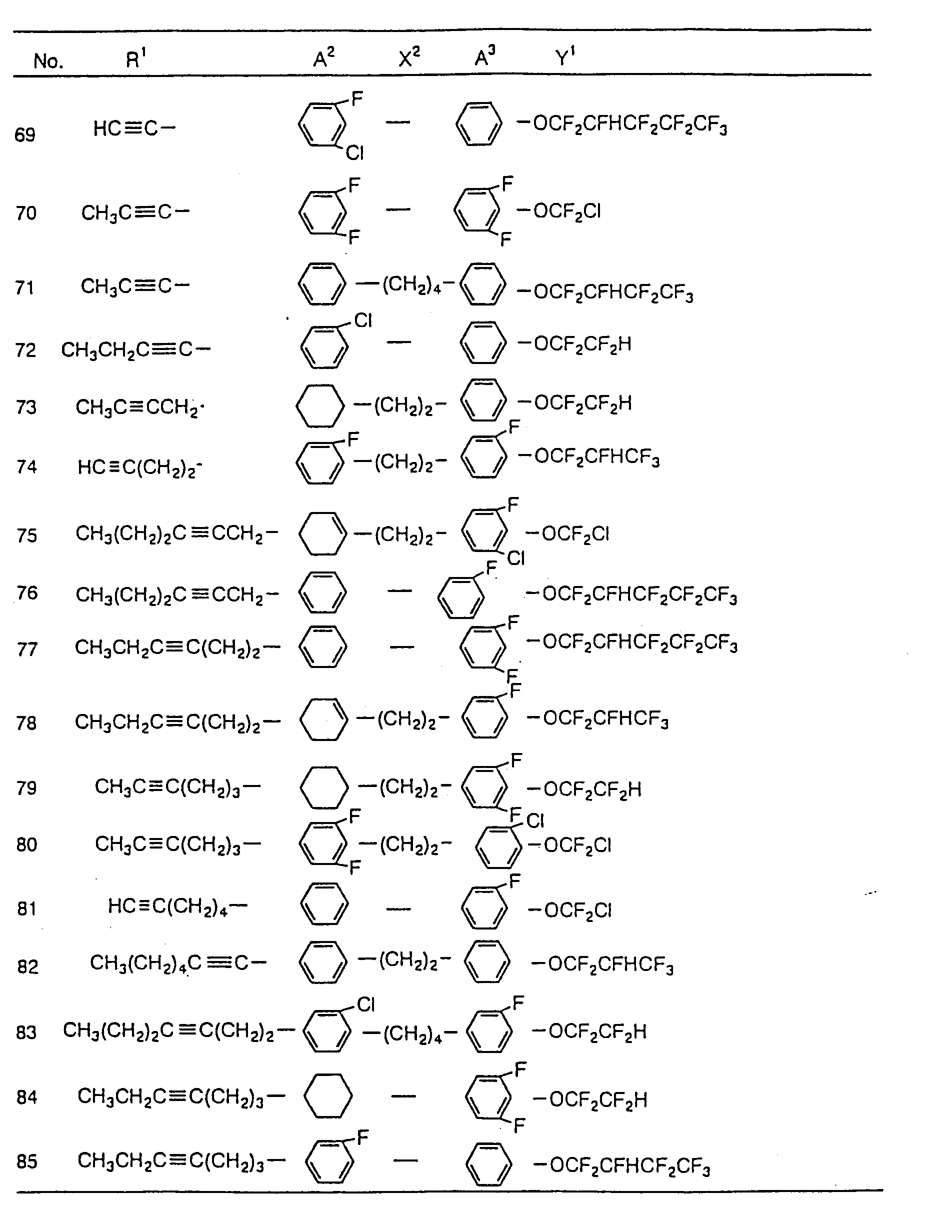

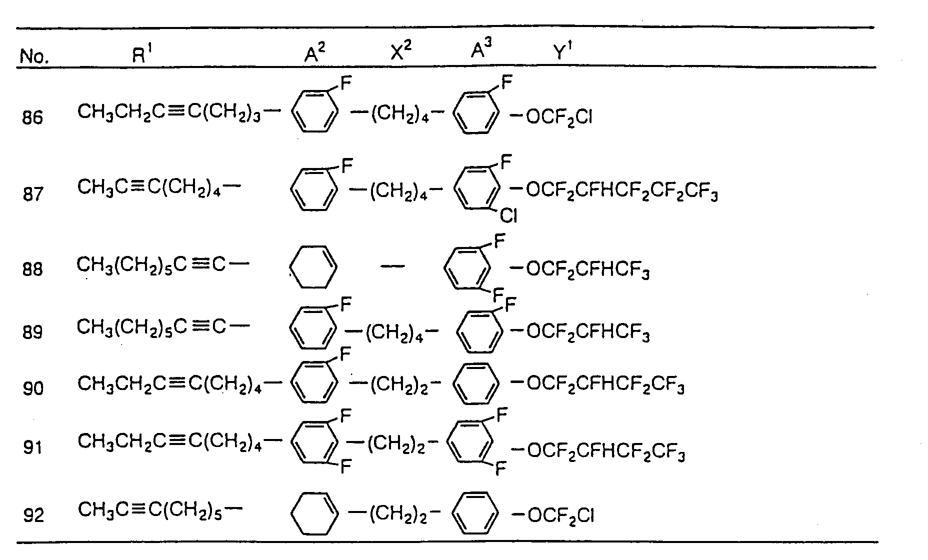

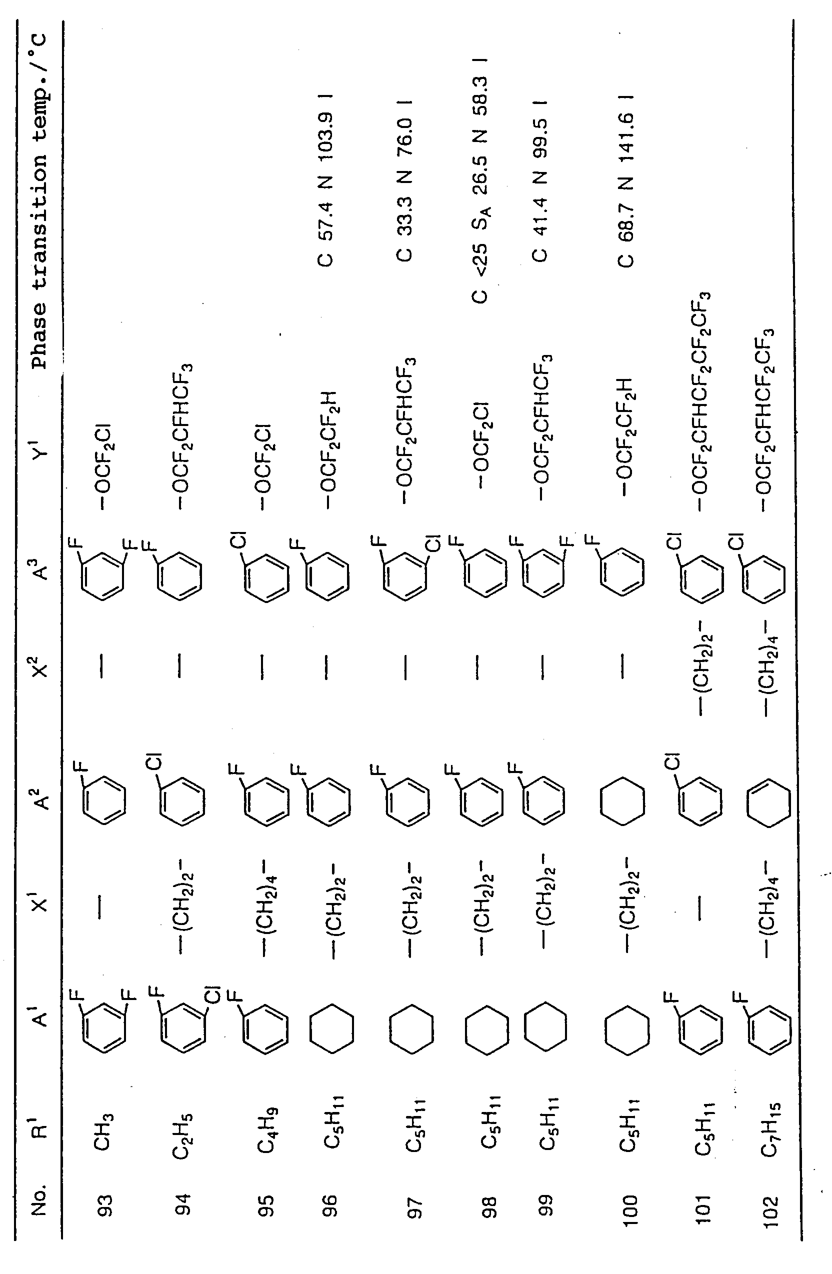

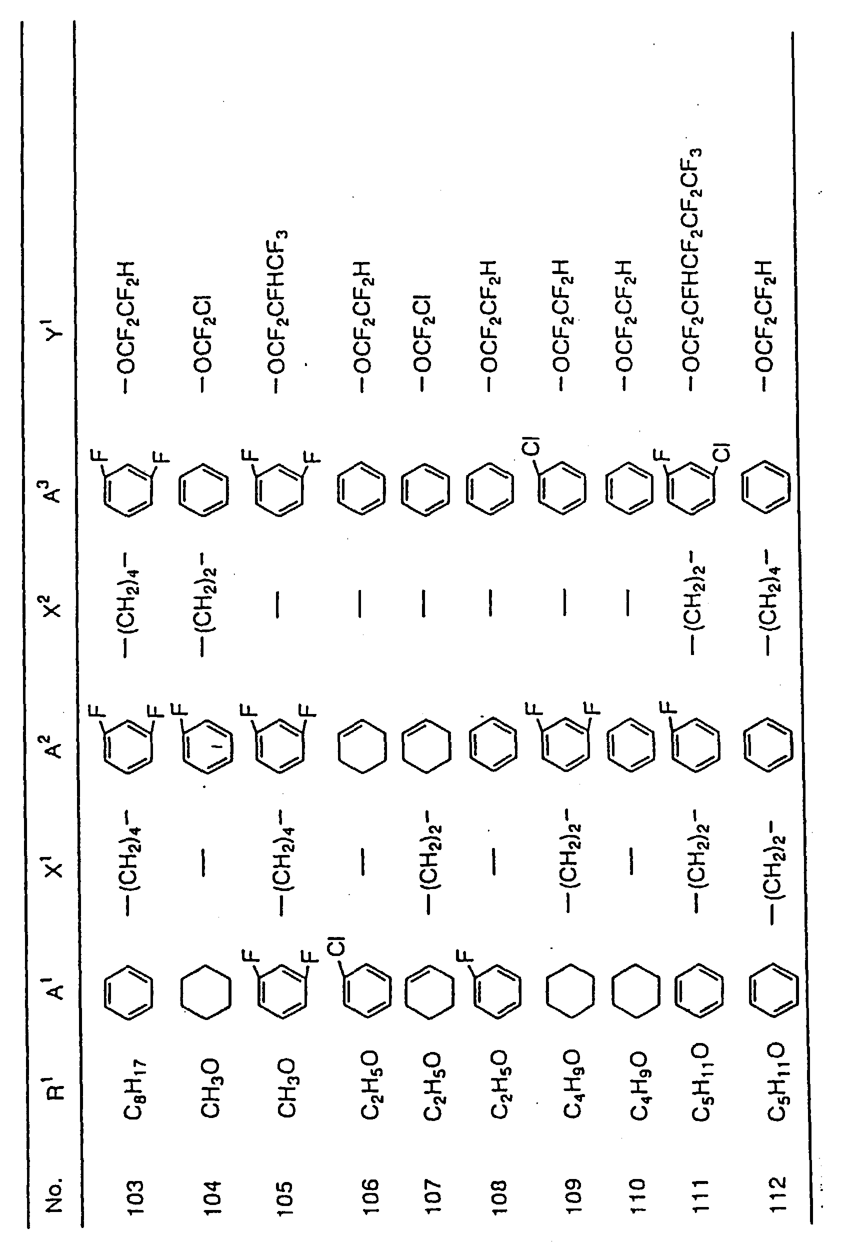

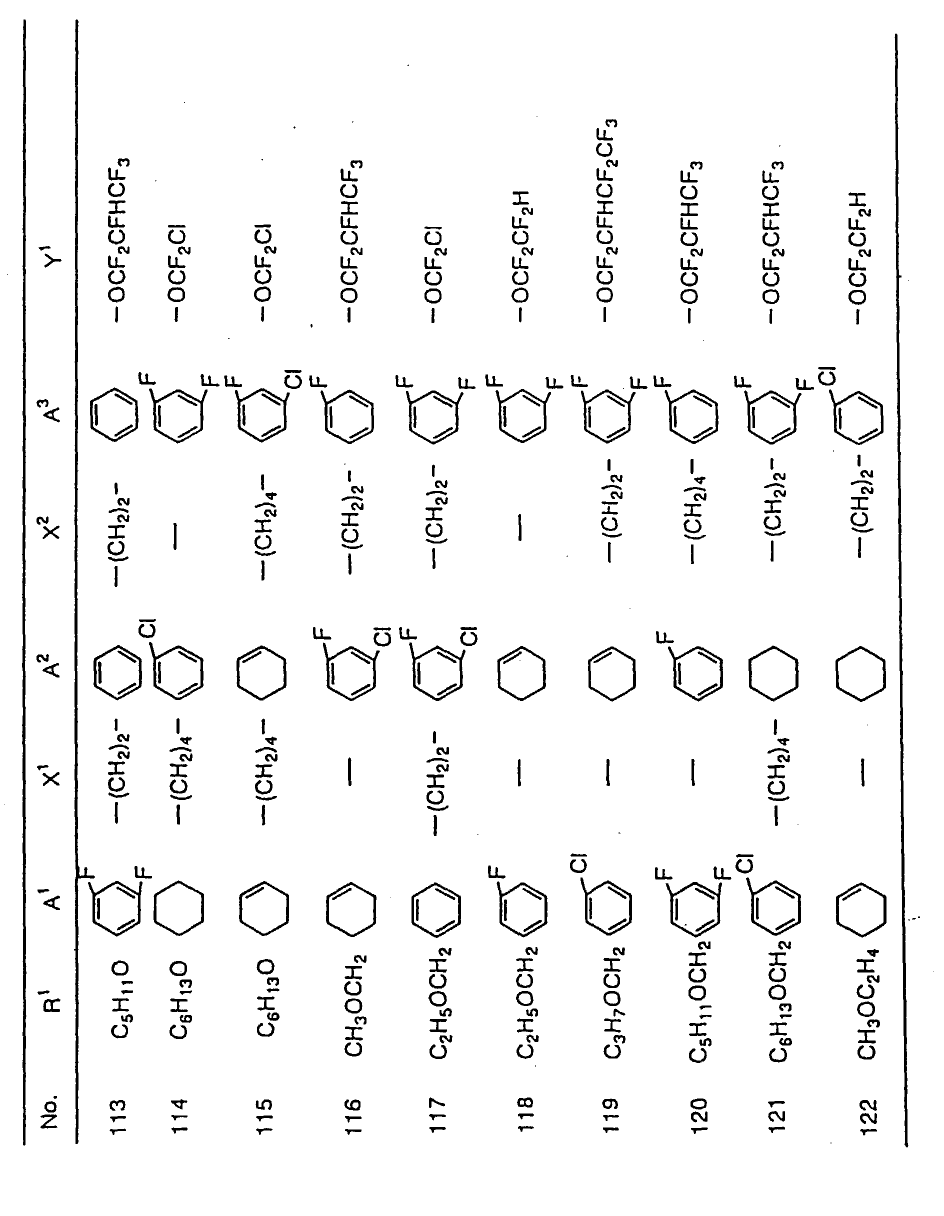

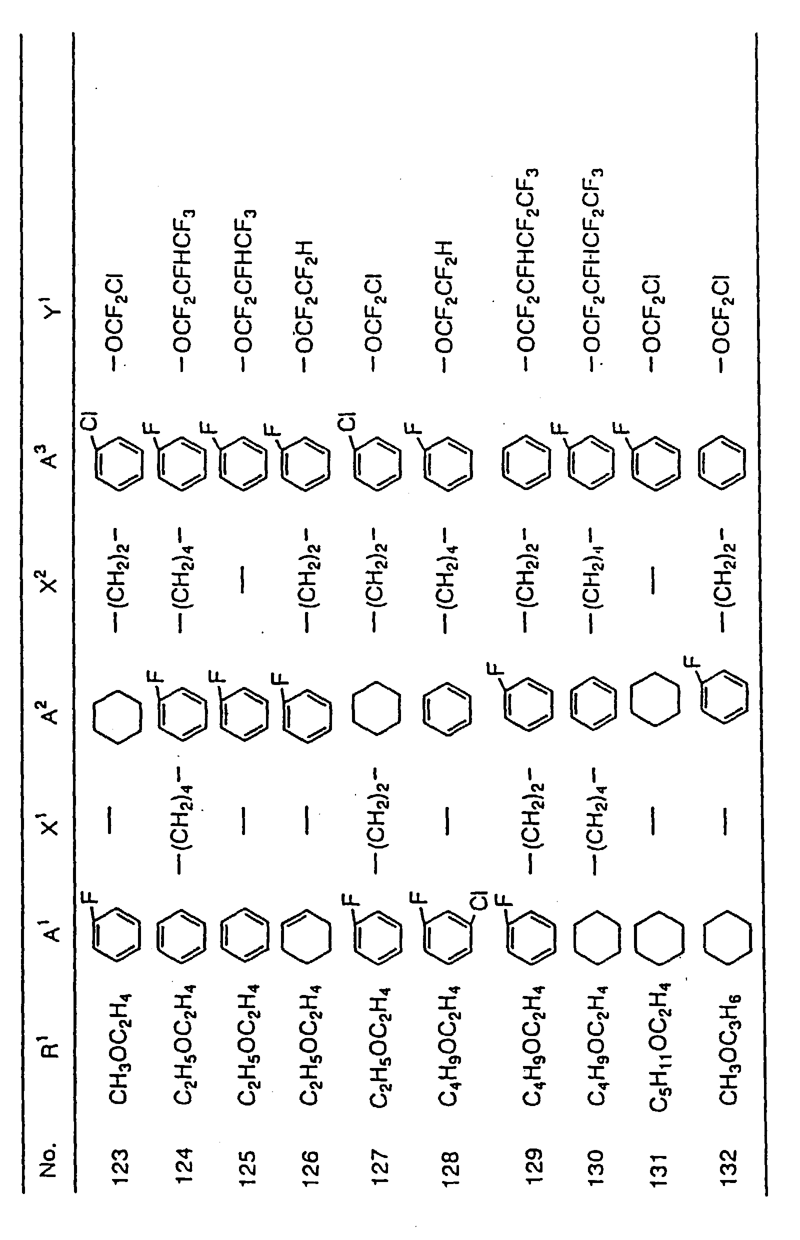

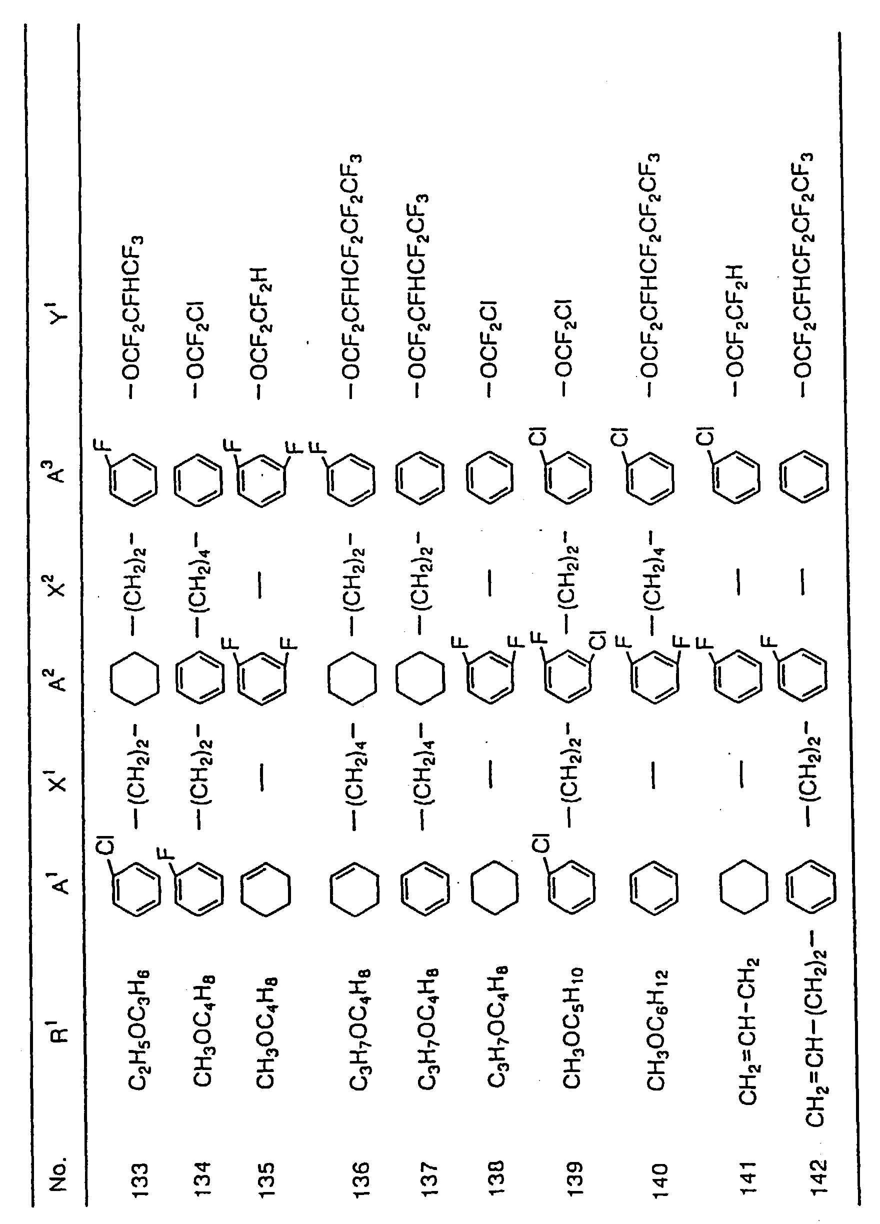

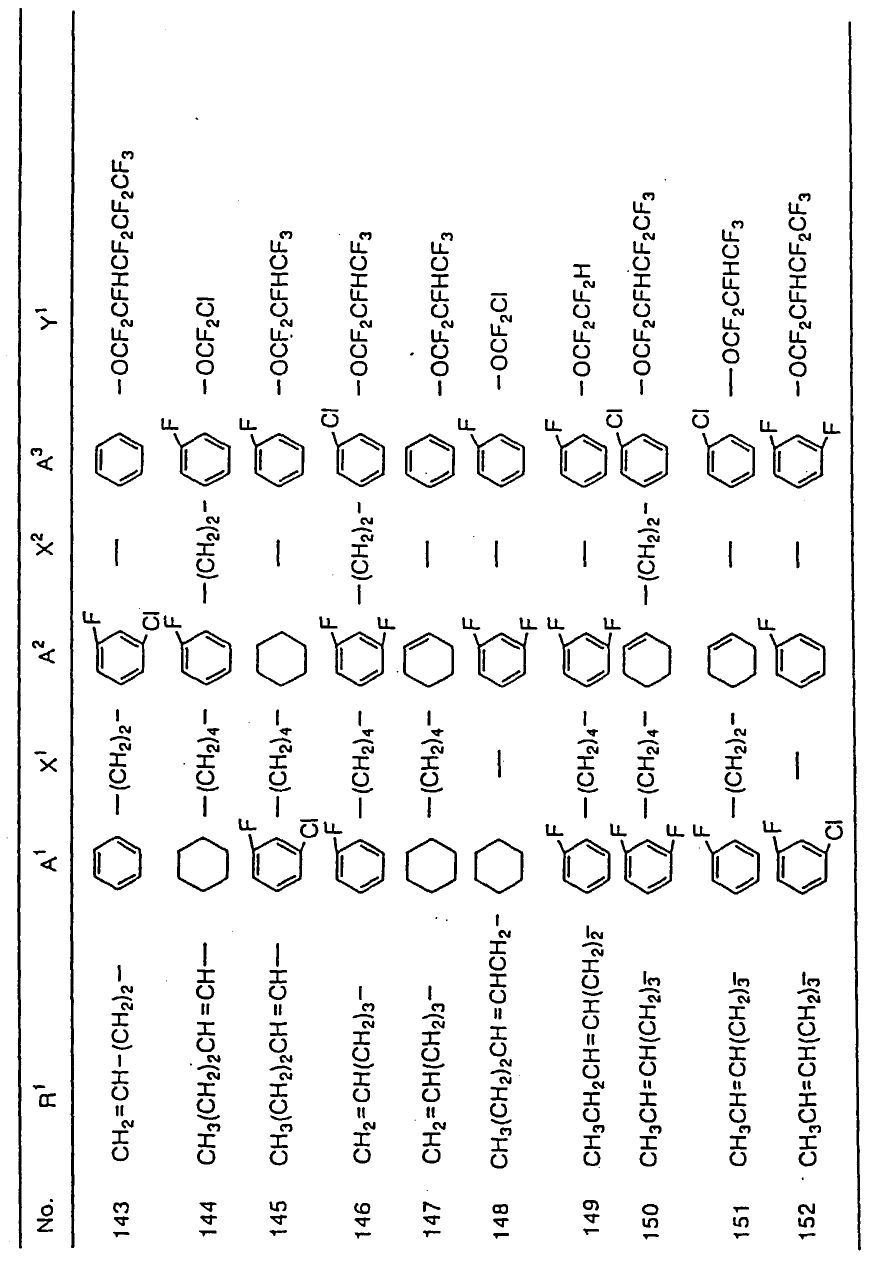

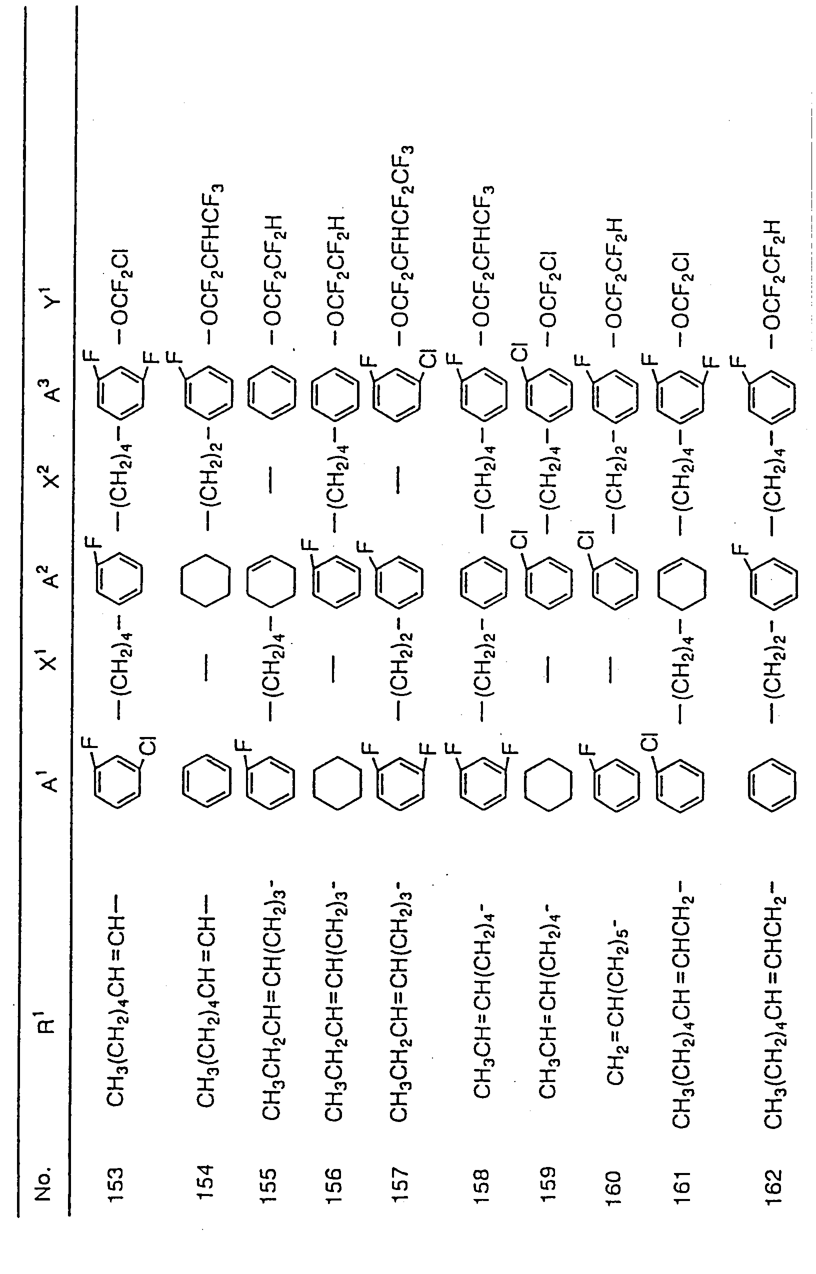

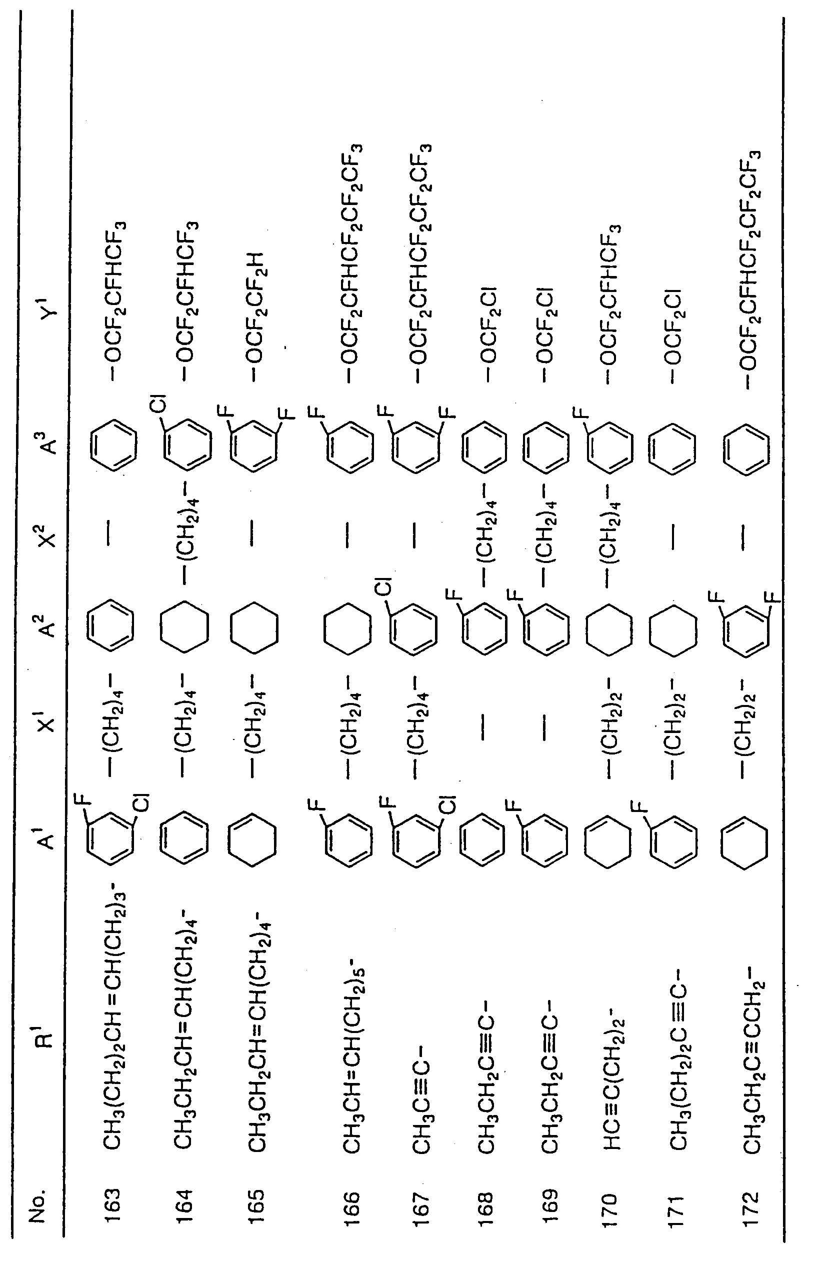

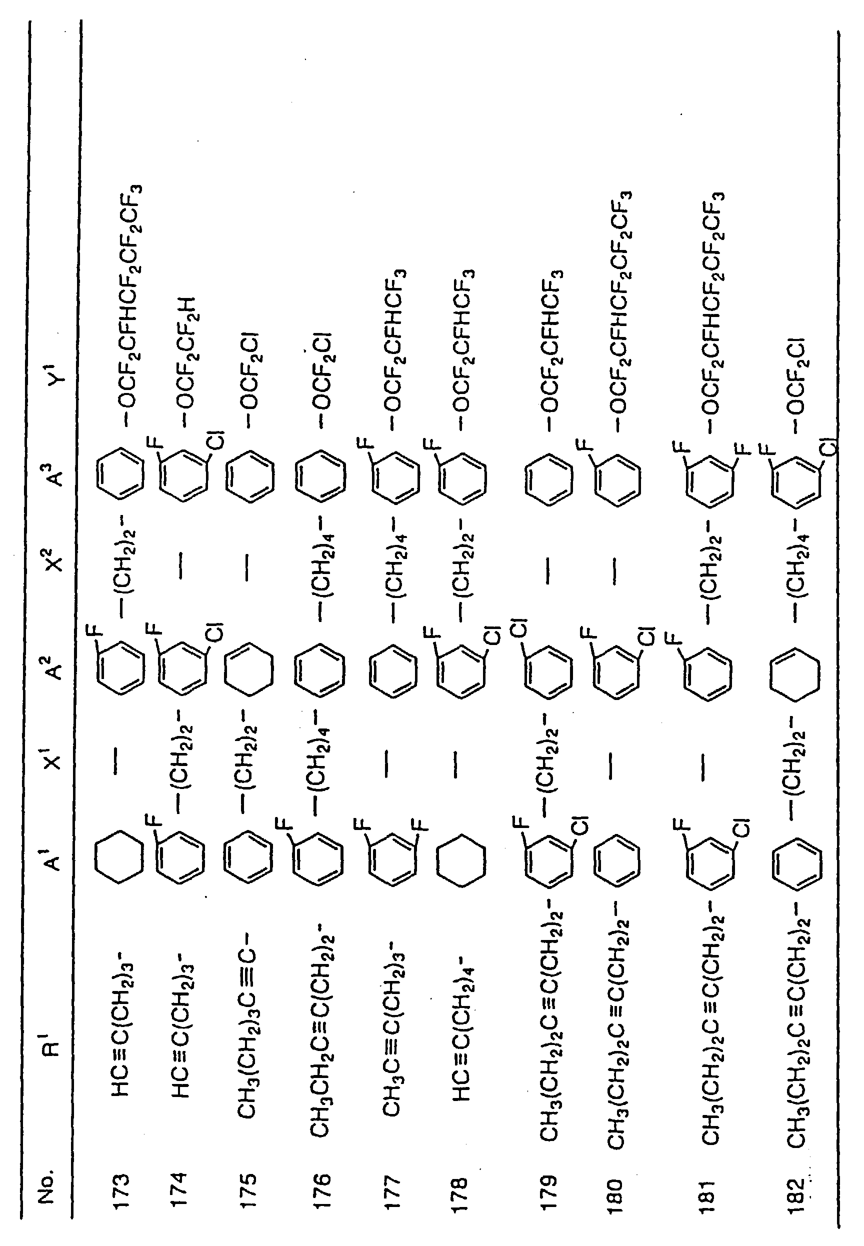

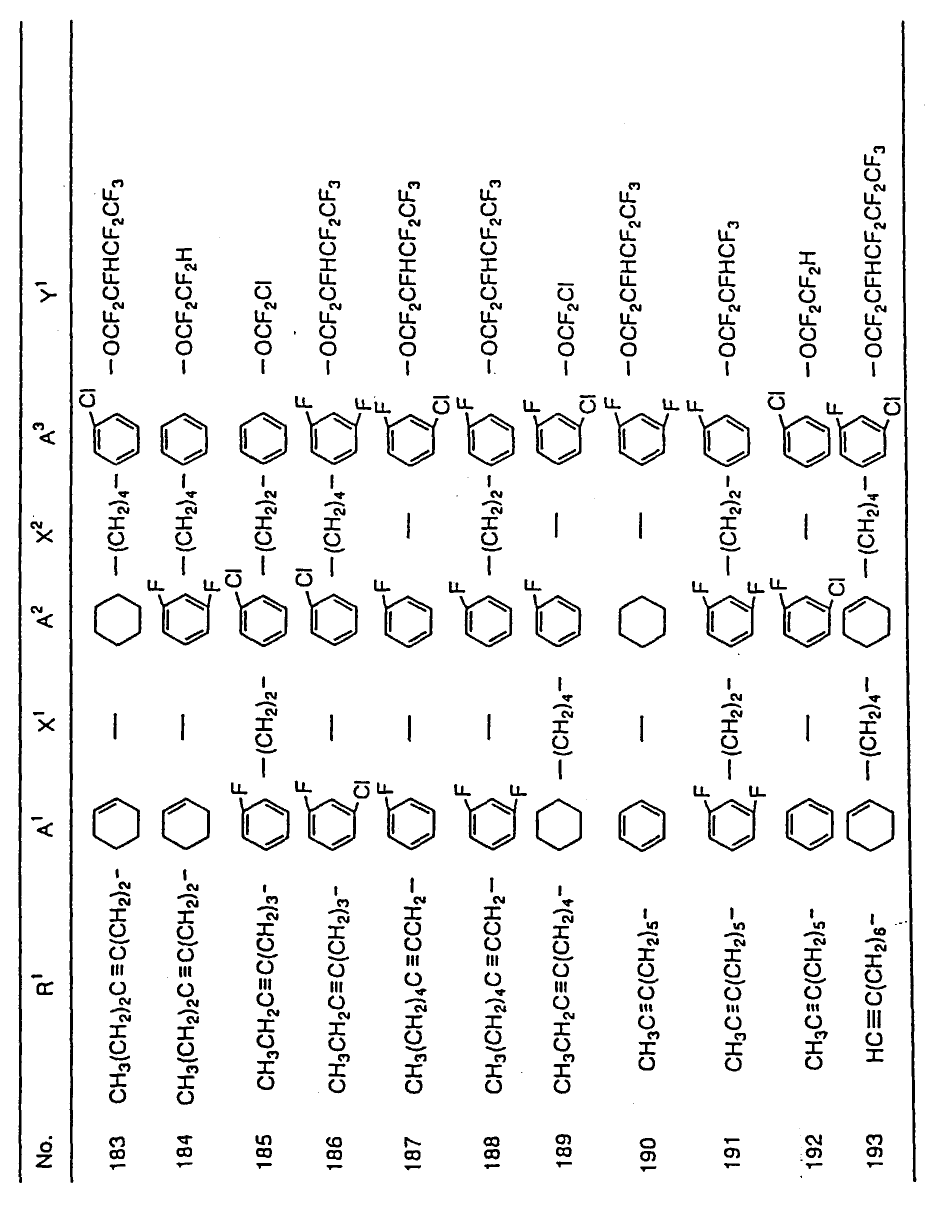

Examples of liquid crystal compositions comprising the liquid crystalline compound of the present invention are shown as Use Examples below.

In each Use Example, compounds are designated by making the group shown in each of the columns of left side terminal group, bonding group, ring structure, or right side terminal group to the symbol shown in the column thereof according to definition indicated in Table 1 below.

Compounds having the same Compound No. in the following Use Examples and Examples described above are the same ones, and the amount of the compounds means "% by weight" unless otherwise specified.

Further, data of characteristics in Use Examples are indicated by T_{NI} (clearing point), η (viscosity: determined at 20°C), Δn (optical anisotropy: determined at 25°C), Δε (dielectric anisotropy: determined at 25 °C), and Vₜₕ (threshold voltage: determined at 25°C).

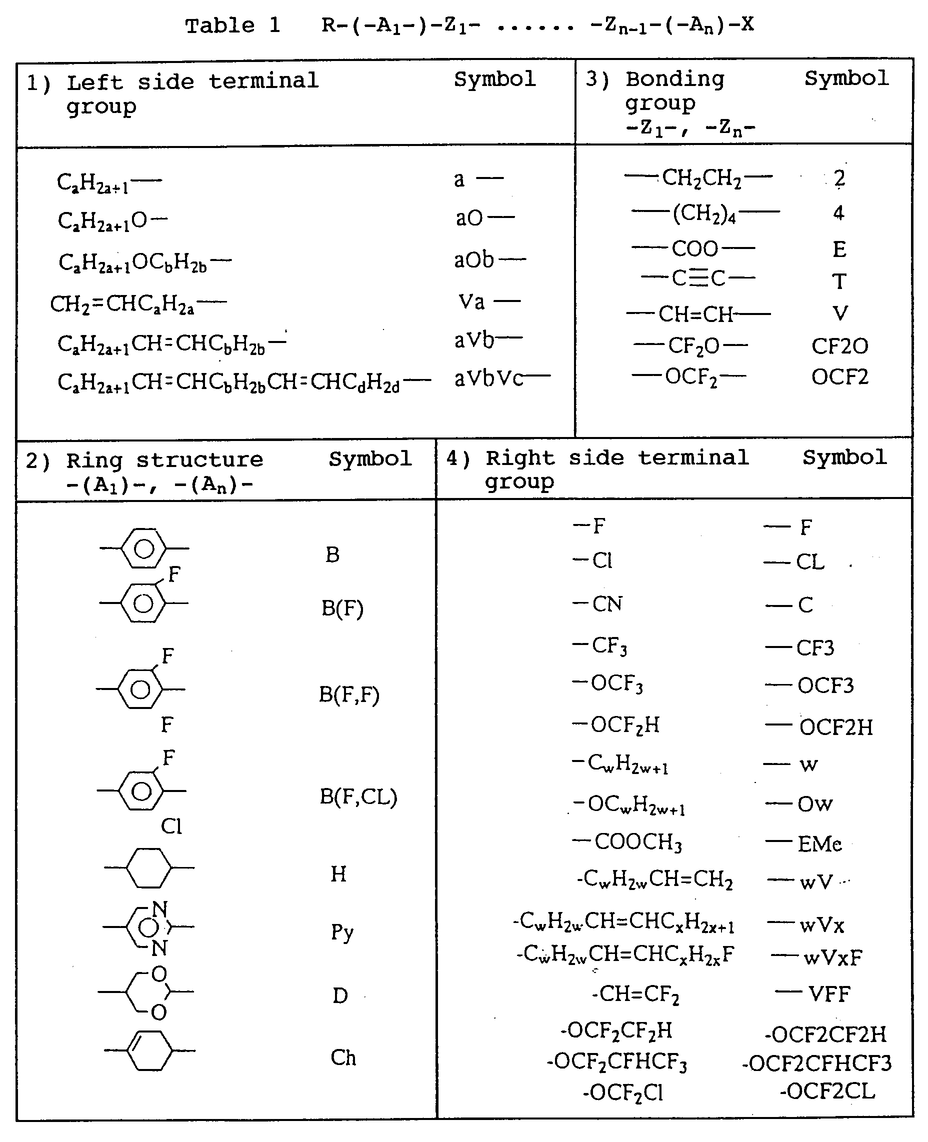

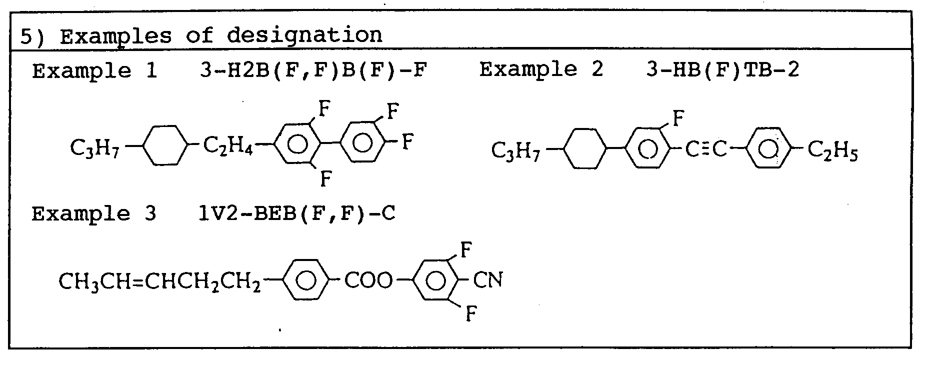

### Example 3 (Use Example 1)

Liquid crystal composition comprising the following compounds in the amount shown below, respectively, was prepared:

| | |
|---|---|
| 5-H2B(F)B(F)-OCF2CF2H (No. 96) | 5.0% |
| 5-H2B(F)B(F,CL)-OCF2CFHCF3 (No. 97) | 5.0% |
| 1V2-BEB(F,F)-C | 5.0% |
| 3-HB-C | 23.0% |
| 1-BTB-3 | 5.0% |
| 2-BTB-1 | 10.0% |
| 3-HH-4 | 11.0% |
| 3-HHB-1 | 11.0% |
| 3-HHB-3 | 7.0% |
| 3-H2BTB-2 | 4.0% |
| 3-H2BTB-3 | 4.0% |
| 3-H2BTB-4 | 4.0% |
| 3-HB(F)TB-3 | 6.0% |

Characteristics of this composition were determined to find to be as follows:
T_{NI} = 85.7°C
η = 19.1 mPa·s
Δn = 0.154
Δε = 7.0
Vₜₕ = 2.12 V

### Example 4 (Use Example 2)

Liquid crystal composition comprising the following compounds in the amount shown below, respectively, was prepared:

| | |
|---|---|
| 5-H2B(F)B(F)-OCF2CL (No. 98) | 5.0% |
| 5-H2B(F)B(F,F)-OCF2CFHCF3 (No. 99) | 5.0% |
| V2-HB-C | 10.0% |
| 1V2-HB-C | 10.0% |
| 3-HB-C | 24.0% |
| 3-HB(F)-C | 5.0% |
| 2-BTB-1 | 2.0% |
| 3-HH-4 | 8.0% |
| 3-HH-VFF | 6.0% |
| 3-HHB-C | 6.0% |
| 3-HB(F)TB-2 | 5.0% |
| 3-H2BTB-2 | 5.0% |
| 3-H2BTB-3 | 5.0% |
| 3-H2BTB-4 | 4.0% |

Characteristics of this composition were determined to find to be as follows:
T_{NI} = 82.7°C
η = 20.9 mPa·s
Δn = 0.147
Δε = 8.7
Vₜₕ = 2.00 V

### Example 5 (Use Example 3)

Liquid crystal composition comprising the following compounds in the amount shown below, respectively, was prepared:

| | |
|---|---|
| 5-H2B(F)B(F)-OCF2CF2H (No. 96) | 4.0% |
| 5-H2HB(F)-OCF2CF2H (No. 100) | 4.0% |
| 2O1-BEB(F)-C | 5.0% |
| 3O1-BEB(F)-C | 15.0% |
| 4O1-BEB(F)-C | 13.0% |
| 5O1-BEB(F)-C | 13.0% |
| 2-HHB(F)-C | 11.0% |
| 3-HHB(F)-C | 15.0% |
| 3-HB(F)TB-2 | 4.0% |
| 3-HB(F)TB-3 | 4.0% |
| 3-HB(F)TB-4 | 4.0% |
| 3-HHB-1 | 4.0% |
| 3-HHB-O1 | 4.0% |

Characteristics of this composition were determined to find to be as follows:
T_{NI} = 88.7°C
η = 88.0 mPa·s
Δn = 1.148
Δε = 30.7
Vₜₕ = 0.88 V

### Example 6 (Use Example 4)

Liquid crystal composition comprising the following compounds in the amount shown below, respectively, was prepared:

| | |
|---|---|
| 5-H2B(F)B(F)-OCF2CF2H (No. 96) | 5.0% |
| 5-H2B(F)B(F,CL)-OCF2CFHCF3 (No. 97) | 5.0% |
| 5-H2HB(F)-OCF2CF2H (No. 100) | 5.0% |
| 5-PyB-F | 4.0% |
| 3-PyB(F)-F | 4.0% |
| 2-BB-C | 5.0% |
| 4-BB-C | 4.0% |
| 5-BB-C | 5.0% |
| 2-PyB-2 | 2.0% |
| 4-PyB-2 | 2.0% |
| 6-PyB-O5 | 3.0% |
| 6-PyB-O6 | 3.0% |
| 6-PyB-O8 | 3.0% |
| 3-PyBB-F | 6.0% |
| 5-PyBB-F | 6.0% |
| 3-HHB-1 | 6.0% |
| 3-HHB-3 | 8.0% |
| 2-H2BTB-3 | 4.0% |
| 2-H2BTB-4 | 5.0% |
| 3-H2BTB-2 | 5.0% |
| 3-H2BTB-3 | 5.0% |
| 3-H2BTB-4 | 5.0% |

Characteristics of this composition were determined to find to be as follows:
T_{NI} = 92.9°C
η = 39.7 mPa·s
Δn = 0.189
Δε = 6.1
Vₜₕ = 2.30 V

### Example 7 (Use Example 5)

Liquid crystal composition comprising the following compounds in the amount shown below, respectively, was prepared:

| | |
|---|---|
| 5-H2B(F)B(F)-OCF2CL (No. 98) | 5.0% |
| 5-H2B(F)B(F,F)-OCF2CFHCF3 (No. 99) | 5.0% |
| 5-H2HB(F)-OCF2CF2H (No. 100) | 5.0% |
| 3-DB-C | 10.0% |
| 4-DB-C | 10.0% |
| 2-BEB-C | 12.0% |
| 3-BEB-C | 4.0% |
| 3-PyB(F)-F | 6.0% |
| 4-HEB-O2 | 6.0% |
| 5-HEB-O1 | 6.0% |
| 5-HEB-O2 | 4.0% |
| 5-HEB-5 | 5.0% |
| 4-HEB-5 | 5.0% |
| 1O-BEB-2 | 4.0% |
| 3-HHB-1 | 6.0% |
| 3-HHEBB-C | 3.0% |
| 3-HBEBB-C | 2.0% |
| 5-HBEBB-C | 2.0% |

Characteristics of this composition were determined to find to be as follows:
T_{NI} = 69.5°C
η = 44.1 mPa·s
Δn = 0.121
Δε = 12.0
Vₜₕ = 1.25 V

### Example 8 (Use Example 6)

Liquid crystal composition comprising the following compounds in the amount shown below, respectively, was prepared:

| | |
|---|---|
| 5-H2B(F)B(F,CL)-OCF2CFHCF3 (No. 97) | 3.0% |
| 5-H2B(F)B(F)-OCF2CL (No. 98) | 3.0% |
| 5-H2B(F)B(F,F)-OCF2CFHCF3 (No. 99) | 3.0% |
| 3-HB-C | 18.0% |
| 5-HB-C | 3.0% |
| 1O1-HB-C | 10.0% |
| 3-HB(F)-C | 7.0% |
| 2-PyB-2 | 2.0% |
| 3-PyB-2 | 2.0% |
| 4-PyB-2 | 2.0% |
| 1O1-HH-3 | 7.0% |
| 2-BTB-O1 | 7.0% |
| 3-HHB-1 | 7.0% |
| 3-HHB-F | 4.0% |
| 3-HHB-O1 | 4.0% |
| 3-HHB-3 | 2.0% |
| 3-H2BTB-2 | 3.0% |
| 3-H2BTB-3 | 3.0% |
| 2-PyBH-3 | 4.0% |
| 3-PyBH-3 | 3.0% |
| 3-PyBB-2 | 3.0% |

Characteristics of this composition were determined to find to be as follows:
T_{NI} = 78.0°C
η = 22.9 mPa·s
Δn = 0.139
Δε = 8.1
Vₜₕ = 1.75 V

### Example 9 (Use Example 7)

Liquid crystal composition comprising the following compounds in the amount shown below, respectively, was prepared:

| | |
|---|---|
| 5-H2B(F)B(F)-OCF2CL (No. 98) | 10.0% |
| 2O1-BEB(F)-C | 5.0% |
| 3O1-BEB(F)-C | 9.0% |
| 5O1-BEB(F)-C | 4.0% |
| 1V2-BEB(F,F)-C | 10.0% |
| 3-HH-EMe | 10.0% |
| 3-HB-O2 | 18.0% |
| 3-HHEB-F | 3.0% |
| 5-HHEB-F | 3.0% |
| 3-HBEB-F | 4.0% |
| 2O1-HBEB(F)-C | 2.0% |
| 3-HB(F)EB(F)-C | 2.0% |
| 3-HBEB(F,F)-C | 2.0% |
| 3-HHB-F | 4.0% |
| 3-HHB-O1 | 4.0% |
| 3-HHB-3 | 6.0% |
| 3-HEBEB-F | 2.0% |
| 3-HEBEB-1 | 2.0% |

Characteristics of this composition were determined to find to be as follows:
T_{NI} = 72.9°C
η = 37.5 mPa·s
Δn = 0.115
Δε = 23.2
Vₜₕ = 1.00 V

### Example 10 (Use Example 8)

Liquid crystal composition comprising the following compounds in the amount shown below, respectively, was prepared:

| | |
|---|---|
| 5-H2B(F)B(F)-OCF2CF2H (No. 96) | 5.0% |
| 8-B4B(F,F)4B(F,F)-OCF2CF2H (No. 103) | 5.0% |
| 5-BEB(F)-C | 5.0% |
| V-HB-C | 6.0% |
| 5-PyB-C | 6.0% |
| 4-BB-3 | 11.0% |
| 3-HH-2V | 10.0% |
| 5-HH-V | 11.0% |
| V-HHB-1 | 7.0% |
| V2-HHB-1 | 10.0% |
| 3-HHB-1 | 9.0% |
| 1V2-HBB-2 | 10.0% |
| 3-HHEBH-3 | 5.0% |

Characteristics of this composition were determined to find to be as follows:
T_{NI} = 87.4°C
η = 18.8 mPa·s
Δn = 0.116
Δε = 4.5
Vₜₕ = 2.40 V

### Example 11 (Use Example 9)

Liquid crystal composition comprising the following compounds in the amount shown below, respectively, was prepared:

| | |
|---|---|
| 5-H2B(F)B(F,CL)-OCF2CFHCF3 (No. 97) | 10.0% |
| 5-H2HB(F)-OCF2CF2H (No. 100) | 10.0% |
| 2O1-BEB(F)-C | 5.0% |
| 3O1-BEB(F)-C | 7.0% |
| 5O1-BEB(F)-C | 4.0% |
| 1V2-BEB(F,F)-C | 16.0% |
| 3-HB-O2 | 10.0% |
| 3-HH-4 | 3.0% |
| 3-HHB-F | 3.0% |
| 3-HHB-O1 | 4.0% |
| 3-HBEB-F | 4.0% |
| 5-HHEB-F | 7.0% |
| 3-H2BTB-2 | 4.0% |
| 3-H2BTB-3 | 4.0% |
| 3-H2BTB-4 | 4.0% |
| 3-HB(F)TB-2 | 5.0% |

Characteristics of this composition were determined to find to be as follows:
T_{NI} = 86.8°C
η = 49.6 mPa·s
Δn = 0.140
Δε = 26.9
Vₜₕ = 1.02 V

### Example 12 (Use Example 10)

Liquid crystal composition comprising the following compounds in the amount shown below, respectively, was prepared:

| | |
|---|---|
| 5-H2HB(F)-OCF2CF2H (No. 100) | 3.0% |
| 5-H2B(F)-OCF2CFHCF3 (No. 5) | 3.0% |
| 8-B4B(F,F)4B(F,F)-OCF2CF2H (No. 103) | 3.0% |
| 2-BEB-C | 10.0% |
| 3-BEB-C | 4.0% |
| 4-BEB-C | 6.0% |
| 3-HB-C | 28.0% |
| 3-HEB-O4 | 5.0% |
| 4-HEB-O2 | 8.0% |
| 5-HEB-O1 | 8.0% |
| 3-HEB-O2 | 6.0% |
| 5-HEB-O2 | 5.0% |
| 3-HHB-1 | 7.0% |
| 3-HHB-O1 | 4.0% |

Characteristics of this composition were determined to find to be as follows:
T_{NI} = 60.8°C
η = 27.6 mPa·s
n = 0.113
Δε = 9.8
Vₜₕ = 1.38 V

### Example 13 (Use Example 11)

Liquid crystal composition comprising the following compounds in the amount shown below, respectively, was prepared:

| | |
|---|---|
| 5-H2B(F)B(F)-OCF2CF2H (No. 96) | 3.0% |
| 5-H2B(F)B(F,CL)-OCF2CFHCF3 (No. 97) | 3.0% |
| 5-H2B(F)B(F,F)-OCF2CFHCF3 (No. 99) | 3.0% |
| 2-BEB-C | 10.0% |
| 5-BB-C | 8.0% |
| 7-BB-C | 7.0% |
| 1-BTB-3 | 7.0% |
| 2-BTB-1 | 10.0% |
| 1O-BEB-2 | 10.0% |
| 1O-BEB-5 | 12.0% |
| 2-HHB-1 | 4.0% |
| 3-HHB-F | 4.0% |
| 3-HHB-1 | 7.0% |
| 3-HHB-O1 | 4.0% |
| 3-HHB-3 | 8.0% |

Characteristics of this composition were determined to find to be as follows:
T_{NI} = 65.7°C
η = 24.6 mPa·s
Δn = 0.156
Δε = 6.3
Vₜₕ = 1.80 V

### Example 14 (Use Example 12)

Liquid crystal composition comprising the following compounds in the amount shown below, respectively, was prepared:

| | |
|---|---|
| 5-H2B(F)B(F)-OCF2CF2H (No. 96) | 10.0% |
| 5-H2B(F)B(F,CL)-OCF2CFHCF3 (No. 97) | 10.0% |
| 5-H2HB(F)-OCF2CF2H (No. 100) | 10.0% |
| 2-HHB(F)-F | 10.0% |
| 3-HHB(F)-F | 17.0% |
| 5-HHB(F)-F | 6.0% |
| 2-H2HB(F)-F | 10.0% |
| 3-H2HB(F)-F | 5.0% |
| 5-H2HB(F)-F | 10.0% |
| 2-HBB(F)-F | 6.0% |
| 3-HBB(F)-F | 6.0% |

Characteristics of this composition were determined to find to be as follows:
T_{NI} = 99.6°C
η = 35.1 mPa·s
n = 0.092
Δε = 5.6
Vₜₕ = 2.10 V

### Example 15 (Use Example 13)

Liquid crystal composition comprising the following compounds in the amount shown below, respectively, was prepared:

| | |
|---|---|
| 5-H2B(F)B(F)-OCF2CL (No. 98) | 4.0% |
| 5-H2B(F)B(F,F)-OCF2CFHCF3 (No. 99) | 4.0% |
| 5-H2HB(F)-OCF2CF2H (No. 100) | 4.0% |
| 7-HB(F)-F | 5.0% |
| 5-H2B(F)-F | 5.0% |
| 3-HB-O2 | 10.0% |
| 3-HH-4 | 5.0% |
| 2-HHB(F)-F | 10.0% |
| 3-HHB(F)-F | 10.0% |
| 5-HHB(F)-F | 10.0% |
| 3-H2HB(F)-F | 5.0% |
| 2-HBB(F)-F | 3.0% |
| 3-HBB(F)-F | 3.0% |
| 2-H2BB(F)-F | 5.0% |
| 3-HHB-1 | 8.0% |
| 3-HHB-O1 | 5.0% |
| 3-HHB-3 | 4.0% |

Characteristics of this composition were determined to find to be as follows:
T_{NI} = 88.5°C
η = 21.0 mPa·s
Δn = 0.089
Δε = 3.5
Vₜₕ = 2.6 V

### Example 16 (Use Example 14)

Liquid crystal composition comprising the following compounds in the amount shown below, respectively, was prepared:

| | |
|---|---|
| 5-H2B(F)B(F)-OCF2CF2H (No. 96) | 5.0% |
| 5-H2B(F)B(F,F)-OCF2CFHCF3 (No. 99) | 5.0% |
| 5-H2HB(F)-OCF2CF2H (No. 100) | 5.0% |
| 7-HB(F,F)-F | 3.0% |
| 3-HB-O2 | 7.0% |
| 2-HHB(F)-F | 10.0% |
| 3-HHB(F)-F | 10.0% |
| 5-HHB(F)-F | 10.0% |
| 2-HBB(F)-F | 9.0% |
| 3-HBB(F)-F | 9.0% |
| 5-HBB(F)-F | 6.0% |
| 2-HBB-F | 4.0% |
| 3-HBB-F | 4.0% |
| 5-HBB-F | 3.0% |
| 3-HBB(F,F)-F | 5.0% |
| 5-HBB(F,F)-F | 5.0% |

Characteristics of this composition were determined to find to be as follows:
T_{NI} = 82.3°C
η = 27.3 mPa·s
Δn = 0.112
Δε = 6.0
Vₜₕ = 1.95 V

### Example 17 (Use Example 15)

Liquid crystal composition comprising the following compounds in the amount shown below, respectively, was prepared:

| | |
|---|---|
| 5-H2B(F)B(F)-OCF2CF2H (No. 96) | 5.0% |
| 5-H2B(F)B(F,CL)-OCF2CFHCF3 (No. 97) | 5.0% |
| 5-H2B(F)B(F)-OCF2CL (No. 98) | 5.0% |
| 7-HB(F,F)-F | 4.0% |
| 3-H2HB(F,F)-F | 12.0% |
| 4-H2HB(F,F)-F | 10.0% |
| 5-H2HB(F,F)-F | 10.0% |
| 3-HHB(F,F)-F | 10.0% |
| 4-HHB(F,F)-F | 5.0% |
| 3-HH2B(F,F)-F | 10.0% |
| 5-HH2B(F,F)-F | 10.0% |
| 3-HBB(F,F)-F | 7.0% |
| 5-HBB(F,F)-F | 7.0% |

Characteristics of this composition were determined to find to be as follows:
T_{NI} = 73.8°C
η = 32.3 mPa·s
Δn = 0.087
Δε = 8.5
Vₜₕ = 1.60 V

### Example 18 (Use Example 16)

Liquid crystal composition comprising the following compounds in the amount shown below, respectively, was prepared:

| | |
|---|---|
| 5-H2B(F)B(F)-OCF2CF2H (No. 96) | 15.0% |
| 7-HB(F,F)-F | 5.0% |
| 3-H2HB(F,F)-F | 12.0% |
| 4-H2HB(F,F)-F | 10.0% |
| 3-HHB(F,F)-F | 10.0% |
| 3-HBB(F,F)-F | 10.0% |
| 4-HHEB(F,F)-F | 3.0% |
| 5-HHEB(F,F)-F | 3.0% |
| 2-HBEB(F,F)-F | 3.0% |
| 3-HBEB(F,F)-F | 5.0% |
| 5-HBEB(F,F)-F | 3.0% |
| 3-HDB(F,F)-F | 15.0% |
| 3-HHBB(F,F)-F | 6.0% |

Characteristics of this composition were determined to find to be as follows:
T_{NI} = 76.0°C
η = 36.5 mPa·s
n = 0.094
Δε = 12.6
Vₜₕ = 1.43 V

### Example 19 (Use Example 17)

Liquid crystal composition comprising the following compounds in the amount shown below, respectively, was prepared:

| | |
|---|---|
| 5-H2HB(F)-OCF2CF2H (No. 100) | 5.0% |
| 5-H2B(F)-OCF2CFHCF3 (No. 5) | 5.0% |
| 8-B4B(F,F)4B(F,F)-OCF2CF2H (No. 103) | 5.0% |
| 3-HB-CL | 10.0% |
| 7-HB-CL | 4.0% |
| 1O1-HH-5 | 5.0% |
| 2-HBB(F)-F | 8.0% |
| 3-HBB(F)-F | 8.0% |
| 5-HBB(F)-F | 8.0% |
| 4-HHB-CL | 8.0% |
| 5-HHB-CL | 8.0% |
| 3-H2HB(F)-CL | 4.0% |
| 3-HBB(F,F)-F | 5.0% |
| 5-H2BB(F,F)-F | 9.0% |
| 3-HB(F)VB-2 | 4.0% |
| 3-HB(F)VB-3 | 4.0% |

Characteristics of this composition were determined to find to be as follows:
T_{NI} = 92.3°C
η = 23.0 mPa·s
Δn = 0.126
Δε = 4.7
Vₜₕ = 2.35 V

### Example 20 (Use Example 18)

Liquid crystal composition comprising the following compounds in the amount shown below, respectively, was prepared:

| | |
|---|---|
| 5-H2B(F)B(F)-OCF2CF2H (No. 96) | 5.0% |
| 5-H2B(F)B(F)-OCF2CL (No. 98) | 5.0% |
| 5-H2HB(F)-OCF2CF2H (No. 100) | 5.0% |
| 3-HHB(F,F)-F | 9.0% |
| 3-H2HB(F,F)-F | 8.0% |
| 4-H2HB(F,F)-F | 8.0% |
| 3-HBB(F,F)-F | 21.0% |
| 5-HBB(F,F)-F | 15.0% |
| 3-H2BB(F,F)-F | 10.0% |
| 5-HHBB(F,F)-F | 3.0% |
| 3-HH2BB(F,F)-F | 3.0% |
| 5-HHEBB-F | 2.0% |
| 1O1-HBBH-4 | 3.0% |
| 1O1-HBBH-5 | 3.0% |

Characteristics of this composition were determined to find to be as follows:
T_{NI} = 97.0°C
η = 36.3 mPa·s
Δn = 0.119
Δε = 8.9
Vₜₕ = 1.77 V

### Example 21 (Use Example 19)

Liquid crystal composition comprising the following compounds in the amount shown below, respectively, was prepared:

| | |
|---|---|
| 5-H2B(F)-OCF2CFHCF3 (No. 5) | 5.0% |
| 8-B4B(F,F)4B(F,F)-OCF2CF2H (No. 103) | 5.0% |
| 5-HB-F | 12.0% |
| 6-HB-F | 4.0% |
| 7-HB-F | 7.0% |
| 2-HHB-OCF3 | 7.0% |
| 3-HHB-OCF3 | 11.0% |
| 4-HHB-OCF3 | 7.0% |
| 5-HHB-OCF3 | 5.0% |
| 3-HH2B-OCF3 | 4.0% |
| 5-HH2B-OCF3 | 4.0% |
| 3-HHB(F,F)-OCF3 | 5.0% |
| 3-HBB(F)-F | 10.0% |
| 5-HBB(F)-F | 5.0% |
| 3-HH2B(F)-F | 3.0% |
| 3-HB(F)BH-3 | 3.0% |
| 5-HBBH-3 | 3.0% |

Characteristics of this composition were determined to find to be as follows:
T_{NI} = 83.8°C
η = 17.2 mPa·s
Δn = 0.095
Δε = 5.1
Vₜₕ = 2.29 V

### Example 22 (Use Example 20)

Liquid crystal composition comprising the following compounds in the amount shown below, respectively, was prepared:

| | |
|---|---|
| 5-H2B(F)B(F,F)-OCF2CFHCF3 (No. 99) | 10.0% |
| 5-H4HB(F,F)-F | 7.0% |
| 5-H4HB-OCF3 | 10.0% |
| 3-H4HB(F,F)-CF3 | 8.0% |
| 5-H4HB(F,F)-CF3 | 5.0% |
| 3-HB-CL | 6.0% |
| 5-HB-CL | 4.0% |
| 2-H2BB(F)-F | 5.0% |
| 3-H2BB(F)-F | 10.0% |
| 5-HVHB(F,F)-F | 5.0% |
| 3-HHB-OCF3 | 5.0% |
| 3-H2HB-OCF3 | 5.0% |
| V-HHB(F)-F | 5.0% |
| 3-HChB(F)-F | 5.0% |
| 5-HHEB-OCF3 | 2.0% |
| 3-HBEB(F,F)-F | 5.0% |
| 5-HH-V2F | 3.0% |

Characteristics of this composition were determined to find to be as follows:
T_{NI} = 71.4°C
η = 29.2 mPa·s
Δn = 0.093
Δε = 8.2
Vₜₕ = 1.75 V

### Example 23 (Use Example 21)

Liquid crystal composition comprising the following compounds in the amount shown below, respectively, was prepared:

| | |
|---|---|
| 5-H2B(F)B(F)-OCF2CF2H (No. 96) | 5.0% |
| 5-H2B(F)B(F,CL)-OCF2CFHCF3 (No. 97) | 5.0% |
| 5-H2B(F)B(F)-OCF2CL (No. 98) | 5.0% |
| 5-H2B(F)B(F,F)-OCF2CFHCF3 (No. 99) | 5.0% |
| 2-HHB(F)-F | 2.0% |
| 3-HHB(F)-F | 2.0% |
| 5-HHB(F)-F | 2.0% |
| 2-HBB(F)-F | 6.0% |
| 3-HBB(F)-F | 6.0% |
| 5-HBB(F)-F | 5.0% |
| 2-H2BB(F)-F | 9.0% |
| 3-H2BB(F)-F | 9.0% |
| 3-HBB(F,F)-F | 12.0% |
| 5-HBB(F,F)-F | 19.0% |
| 1O1-HBBH-4 | 4.0% |
| 1O1-HBBH-5 | 4.0% |

Characteristics of this composition were determined to find to be as follows:
T_{NI} = 94.8°C
η = 40.8 mPa·s
Δn = 0.132
Δε = 7.3
Vₜₕ = 1.91 V

### Example 24 (Use Example 22)

Liquid crystal composition A1 and A2 were prepared by using ZLI1132 (T_{NI} = 71.7°C, Δε = 11.0) as mother liquid crystal, and dissolving each 15 % by weight of a compound (Compound No. 96) (T_{NI} = 103.9°C) or another compound (Compound No. 97) (T_{NI} = 76.0 ⁰C) of the present invention to the mother liquid crystal, respectively, and their physical characteristics were determined. The results thus obtained were as follows:
A1: T_{NI} = 73.5°C, Δε = 10.6
A2: T_{NI} = 70.0°C, Δε = 10.6

Further, physical property values of the compounds of the Compound Nos. 96 and 97 mentioned above were calculated by extrapolation from the values described above to find to be as follows:
Compound No. 96: T_{NI} = 83.7°C, Δε = 8.3
Compound No. 97: T_{NI} = 60.4°C, Δε = 8.3

### Comparative Example

Two liquid crystal compositions were prepared by the same method as in Example 24 with the exception that compound expressed by the formula (4-33) or (4-34) in both of which R² is C₅H₁₁- was used in place of a Compound (Compound No. 96) or (Compound No. 97), as examples of conventional compounds, and their physical property values were calculated by extrapolation in the same manner as in Example 24 to find to be as follows:
Compound expressed by the formula (4-33): Δε = 7.0
Compound expressed by the formula (4-34): T_{NI} = 61.0°C

From the comparison with Example 24, it can be understood that Δε of the compound expressed by the formula (4-33) is lower than that of both compounds of the present invention, Compound No. 96 and Compound No. 97 even by 1.3, and T_{NI} of the compound expressed by the formula (4-34) is lower than that of the compound of Compound No. 96 of the present invention even by 22.7°C.

As described above, liquid crystalline compounds of the present invention exhibit a particularly high Δε, excellent miscibility with other liquid crystalline compounds, and nematic phase in a wide temperature range, without impairing properties peculiar to liquid crystalline compounds having fluorine atom.

Accordingly, when the liquid crystalline compounds of the present invention are used as component, liquid crystal compositions having excellent characteristics can successfully be provided.

### INDUSTRIAL APPLICABILITY

Liquid crystal display devices fabricated by using a liquid crystal composition comprising the liquid crystalline compound of the present invention can be used for watches, tabletop calculators, various kind of measuring instruments, panels of automobiles, word processors, electronic notebooks, printers, computers, TV sets, or the likes.

## Claims

1. A phenylpolyhaloalkyl ether derivative expressed by the general formula (1) 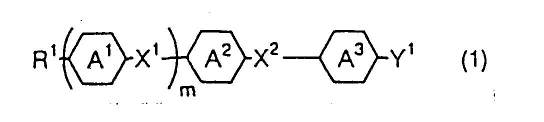 wherein X¹ and X² independently represent 1,2-ethylene group, 1,4-butylene group, or covalent bond provided that in no case are both X¹ and X² simultaneously a covalent bond; R¹ represents an alkyl group having 1 to 20 carbon atoms in which alkyl group methylene group may be replaced by oxygen atom, vinylene group, or ethynylene group; Y¹ represents -OCF₂CFH-(CF₂)ₙ-F (n is 0, 1, 2, or 3) or -OCF₂Cl; rings A¹ and A² independently represent 1,4-cyclohexylene ring, 1,4-cyclohexenylene ring, or 1,4-phenylene ring, A³ represents 1,4-phenylene ring, and the carbon atom in these rings may be replaced by oxygen atom or nitrogen atom, and the hydrogen atom on the rings may be replaced by fluorine atom or chlorine atom, respectively; m is 0 or 1; and each element which constitutes the compound may be its isotope.

2. The phenylpolyhaloalkyl ether derivative according to claim 1 wherein m is 0, and ring A² is 1,4-cyclohexylene ring.

3. The phenylpolyhaloalkyl ether derivative according to claim 1 wherein m is 1, ring A¹ is 1,4-cyclohexylene ring, and X² is a covalent bond.

4. The phenylpolyhaloalkyl ether derivative according to claim 1 wherein m is 1, and X¹ and X² are independently 1,2-ethylene group or 1,4-butylene group.

5. The phenylpolyhaloalkyl ether derivative according to claim 3 wherein ring A² is 2-fluoro-1,4-phenylene group (the carbon atom at position 1 of which links to ring A³) or 2,6-difluoro-1,4-phenylene group (the carbon atom at position 1 of which links to ring A³).

6. The phenylpolyhaloalkyl ether derivative according to claim 3 wherein ring A² is 1,4-cyclohexylene group.

7. A liquid crystal composition comprising at least one phenylpolyhaloalkyl ether derivative defined in any one of claim 1 to 6.

8. A liquid crystal composition comprising, as a first component, at least one phenylpolyhaloalkyl ether derivative defined in any one of claim 1 to 6, and comprising, as a second component, at least one compound selected from the group consisting of the compounds expressed by any one of the general formulas (2), (3), and (4) 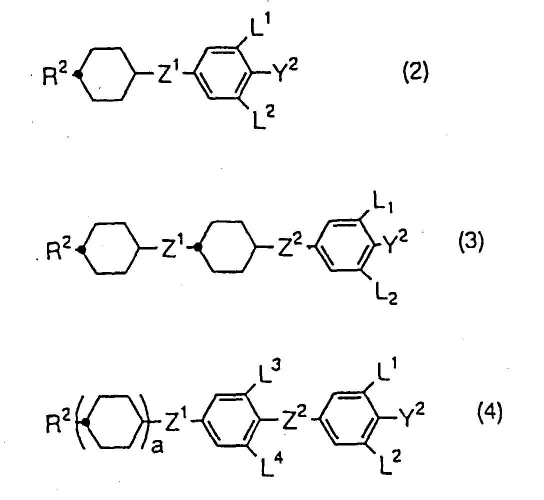 wherein R² represents an alkyl group having 1 to 10 carbon atoms; Y² represents fluorine atom, chlorine atom, trifluoromethoxy group, difluoromethoxy group, trifluoromethyl group, difluoromethyl group, or monofluoromethyl group; L¹, L², L³, and L⁴ independently represent hydrogen atom or fluorine atom; Z¹ and Z² independently represent 1,2-ethylene group, vinylene group, or a covalent bond; and a is 1 or 2.

9. A liquid crystal composition comprising, as a first component, at least one phenylpolyhaloalkyl ether derivative defined in any one of claim 1 to 6, and comprising, as a second component, at least one compound selected from the group consisting of the compounds expressed by any one of the general formulas (5), (6), (7), (8), (9) 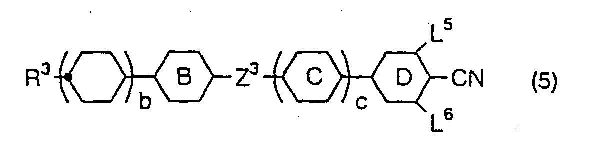 wherein R³ represents fluorine atom, an alkyl group having 1 to 10 carbon atoms, or an alkenyl group having 2 to 10 carbon atoms in which alkyl group or alkenyl group not adjacent one or more methylene groups may be represented by oxygen atom; ring B represents 1,4-cyclohexylene, 1,4-phenylene, or 1,3-dioxane-2,5-diyl: ring C represents 1,4-cyclohexylene, 1,4-phenylene, or pyrimidine-2,5-diyl; ring D represents 1,4-cyclohexylene or 1,4-phenylene; Z³ represents 1,2-ethylene group, oxycarbonyl group, or a covalent bond; L⁵ and L⁶ independently represent hydrogen atom or fluorine atom: and b and c are independently 0 or 1, 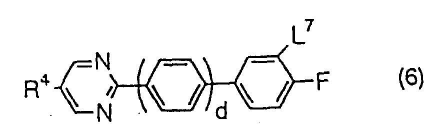 wherein R⁴ represents an alkyl group having 1 to 10 carbon atoms; L⁷ represents hydrogen atom or fluorine atom; and d is 0 or 1, 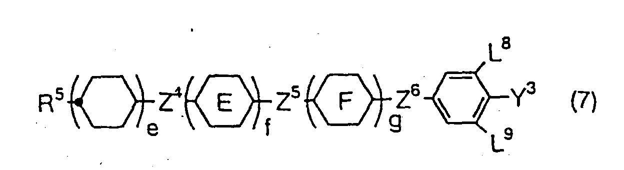 wherein R⁵ represents an alkyl group having 1 to 10 carbon atoms; ring E and ring F independently represent 1,4-cyclohexylene or 1,4-phenylene; Z⁴ and Z⁵ independently represent oxycarbonyl group or a covalent bond; Z⁶ represent oxycarbonyl group or ethynylene group; L⁸ and L⁹ independently represent hydrogen atom or fluorine atom; Y³ represents fluorine atom, trifluoromethoxy group, difluoromethoxy group, trifluoromethyl group, difluoromethyl group, or monofluoromethyl group; and e, f, and g are independently 0 or 1, 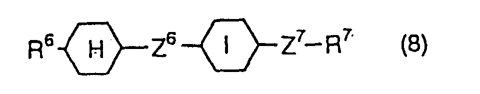 wherein R⁶ and R⁷ independently represent an alkyl group having 1 to 10 carbon atoms or an alkenyl group having 2 to 10 carbon atoms in which alkyl or alkenyl group one or not-adjacent two or more methylene groups may be replaced by oxygen atom; ring H represents 1,4-cyclohexylene, 1,4-phenylene, or pyrimidine-2,5-diyl; ring I represents 1,4-cyclohexylene or 1,4-phenylene; Z⁶ represents ethynylene group, oxycarbonyl group, 1,2-ethylene group, 1-butene-3-ynylene group, or a covalent bond; and Z⁷ represents oxycarbonyl group or a bond, 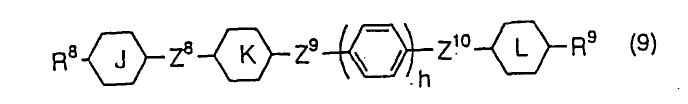 wherein R⁸ and R⁹ independently represent an alkyl group having 1 to 10 carbon atoms or an alkenyl group having 2 to 10 carbon atoms in which alkyl or alkenyl group one or not-adjacent two or more methylene group may be replaced by oxygen atom; ring J represents 1,4-cyclohexylene, 1,4-phenylene, or pyrimidine-2,5-diyl; ring K represents 1,4-cyclohexylene, 1,4-phenylene in which one or more hydrogen atoms may be replaced by fluorine atom, or pyrimidine-2,5-diyl; ring L represents 1,4-cyclohexylene or 1,4-phenylene; Z⁸ and Z¹⁰ independently represent oxycarbonyl group, 1,2-ethylene group, or covalent bond; Z⁹ represents vinylene group, ethynylene group, oxycarbonyl group, or a covalent bond; and h is 0 or 1.

10. A liquid crystal composition comprising, as a first component, at least one phenylpolyhaloalkyl ether derivative defined in any one of claim 1 to 6, and comprising, as a part of a second component, at least one compound selected from the group consisting of the compounds expressed by any one of the general formulas (2), (3), and (4) described in claim 8, and comprising, as another part of the second component, at least one compound selected from the group consisting of the compounds expressed by any one of the general formulas (5), (6), (7), (8), and (9) described in claim 9.

11. A liquid crystal display device fabricated by using the liquid crystal composition defined in claim 7.

12. A liquid crystal display device fabricated by using the liquid crystal composition defined in claim 8.

13. A liquid crystal display device fabricated by using the liquid crystal composition defined in claim 9.

14. A liquid crystal display device fabricated by using the liquid crystal composition defined in claim 10.
